# EUROPEAN PATENT APPLICATION

(11) **EP 1 915 959 A2**
(43) Date of publication of application: **30.04.2008**
(21) Application number: 08002240.3
(22) Date of filing: 08.10.2003
(51) Int. Cl.: A61B 17/88, A61B 17/16, A61B 17/17, A61B 17/58, A61B 17/74

(54) **Apparatus for reducing femoral fractures**

(30) Priority: 08.10.2002 US 266319
(62) Divisional of application: 03256328.0
(71) Applicant: Zimmer Technology, Inc., Warsaw, IN 46580 (US)
(72) Inventor: Thelan, Sarah L., North Manchester, Indiana 46962 (US); Lozier, Anthony J., Warsaw, Indiana 46582 (US); Sisk, Billy N., Claypool, Indiana 46510 (US); Miller, Rick, South Whitley, Indiana 46787 (US); Liberti, Michael Andrew, East Milford, Indiana 46542 (US); Hawkins, Michael E., Columbia City, Indiana 46542 (US)
(74) Representative: Mays, Julie

(57) **Abstract**

An improved method and apparatus for reducing a hip fracture utilizing a minimally invasive procedure which does not require incision of the quadriceps. A femoral implant in accordance with the present invention achieves intramedullary fixation as well as fixation into the femoral head to allow for the compression needed for a femoral fracture to heal. To position the femoral implant of the present invention, an incision is made along the greater trochanter. Because the greater trochanter is not circumferentially covered with muscles, the incision can be made and the wound developed through the skin and fascia to expose the greater trochanter, without incising muscle, including, e.g., the quadriceps. After exposing the greater trochanter, novel instruments of the present invention are utilized to prepare a cavity in the femur extending from the greater trochanter into the femoral head and further extending from the greater trochanter into the intramedullary canal of the femur. After preparation of the femoral cavity, a femoral implant in accordance with the present invention is inserted into the aforementioned cavity in the femur. The femoral implant is thereafter secured in the femur, with portions of the implant extending into and being secured within the femoral head and portions of the implant extending into and being secured within the femoral shaft.

## Description

### CROSS-REFERENCE TO RELATED APPLICATIONS

This is a continuation-in-part of co-pending application Serial No. 10/155,683, filed May 23, 2002.

### BACKGROUND OF THE INVENTION

### 1. Field of the Invention.

The present invention relates to a method and apparatus for treating hip fractures, and, more particularly, to a method and apparatus for reducing femoral fractures utilizing a minimally invasive procedure.

### 2. Description of the Related Art.

Current procedures utilized to reduce hip fractures generally utilize a side plate/hip screw combination, i.e., a bone plate affixed to a lateral aspect of the femur and having a hip screw operably connected thereto, with the hip screw extending into the femoral head. To properly implant a side plate hip screw, a surgeon must dissect an amount of muscle to expose the femur and operably attach the bone plate and hip screw. Typically, the side plate hip screw requires an incision of about 10-12 cm through the quadriceps to expose the femur. While this approach provides surgeons with an excellent view of the bone surface, the underlying damage to soft tissue, including muscle, e.g., the quadriceps can lengthen a patient's rehabilitation time after surgery.

What is needed in the art is a method and apparatus for reducing a hip fracture without requiring incision of soft tissue, including, e.g., the quadriceps.

### SUMMARY OF THE INVENTION

The present invention provides an improved method and apparatus for reducing a hip fracture utilizing a minimally invasive procedure which does not require dissection of the quadriceps. A femoral implant in accordance with the present invention achieves intramedullary fixation as well as fixation into the femoral head to allow for the compression needed for a femoral fracture to heal. The femoral implant of the present invention allows for sliding compression of the femoral fracture. To operably position the femoral implant of the present invention, an incision aligned with the greater trochanter is made and the wound is developed to expose the greater trochanter. The size of the wound developed on the surface is substantially constant throughout the depth of the wound. In one exemplary embodiment of the present invention, the incision through which the femur is prepared and the implant is inserted measures about 2.5 centimeters (1 inch). Because the greater trochanter is not circumferentially covered with muscle, the incision can be made and the wound developed through the skin and fascia to expose the greater trochanter, without incising muscle, including, e.g., the quadriceps. After exposing the greater trochanter, novel instruments of the present invention are utilized to prepare a cavity in the femur extending from the greater trochanter into the femoral head and further extending from the greater trochanter into the intramedullary canal of the femur. After preparation of the femoral cavity, a femoral implant in accordance with the present invention is inserted into the aforementioned cavity in the femur. The femoral implant is thereafter secured in the femur, with portions of the implant extending into and being secured within the femoral head and portions thereof extending into and being secured within the femoral shaft. To allow for sliding compression, the portion of the implant extending into the femoral head is slidable relative to the portion of the implant extending into the femoral shaft.

The femoral implant of the present invention includes a sealed bag having a fill tube positioned therein to provide access to the bag interior so that the implant bag can be filled with material, e.g., bone cement after implantation of the femoral implant within the cavity formed in the femur. The femoral implant of the present invention further includes a lag screw tube placed within the bag of the femoral implant. The bag of the femoral implant is tightly secured to the exterior of the lag screw tube to prevent material injected into the bag from escaping the bag at any point at which the bag contacts the lag screw tube. The lag screw tube is hollow and accommodates a lag screw or other fixation device to be advanced into and secured to the femoral head.

The sealed bag of the femoral implant of the present invention can be, e.g., formed of various films and fabrics. In one exemplary embodiment the bag of the femoral implant of the present invention is formed from an acrylic material, e.g., a woven acrylic material. Because bone cement is an acrylic, if the implant bag is formed of an acrylic material, the bag and the bone cement will achieve an intimate chemical bond. The bag of the femoral implant of the present invention generally comprises a containment device and can be constructed of various materials including films such as, e.g., fiber or fabric reinforced films, or fabrics created by processes such as weaving, knitting, braiding, electrospinning, or hydrospinning. Alternative materials contemplated for the implant bag include various polymers including, e.g., polymethylmethacrylate, polycarbonate, ultra-high molecular weight polyethylene (UHMWPE), low density polyethylene (LDPE), high density polyethylene (HDPE), polyamides, polypropylene, polyester, polyaryletherketone, polysulfone, or polyurethane. Further alternative materials contemplated for the implant bag include fabrics constructed of fibers formed of glass, ceramics, surgical grade stainless steel (e.g., 316L), titanium, or titanium alloys. Moreover, implant bag materials may be coated with, e.g., calcium phosphate, or a bioactive glass coating. Furthermore, the implant bag and filler may be utilized as a delivery mechanism for, e.g., drugs, or growth factors.

In a further embodiment of the present invention, the bag structure of the implant of the present invention comprises a nested bag structure in which an inner bag is filled with a high strength material relative to the material of an outer bag in which the inner bag is placed. The outer bag of this form of the present invention is formed from and filled with a more bioresorbable material relative to the material of construction and fill material of the inner bag.

The femoral implant of the present invention is inserted through an access aperture formed in the greater trochanter and placed within the femoral cavity described hereinabove. The lag screw or other fixation device is thereafter advanced through the lag screw tube and into the cavity formed in the femoral head. The lag screw or other fixation device is then secured to the femoral head. The fill tube is thereafter utilized to fill the femoral implant with, e.g., bone cement to fill the femoral cavity and provide intramedullary fixation and stabilization of the lag screw. In an alternative embodiment of the present invention, bone cement is utilized in lieu of or in addition to lag screw threads to secure a lag screw shaft of an implant of the present invention.

Several different guides and reamers may be utilized in accordance with the present invention to ream the femoral cavity described hereinabove. These novel guides and reamers will be described in detail in the detailed description portion of this document. Generally, the guides and reamers of the present invention are designed to allow for formation of a femoral cavity from the greater trochanter across the femoral neck and into the femoral head as well as from the greater trochanter into the intramedullary canal, with the femoral cavity having exposed access thereto only over the greater trochanter.

The method and apparatus of the current invention advantageously allow for the treatment of a femoral hip fracture in a minimally invasive procedure, which hastens patient recovery.

### BRIEF DESCRIPTION OF THE DRAWINGS

The above-mentioned and other features and advantages of this invention, and the manner of attaining them, will become more apparent and the invention itself will be better understood by reference to the following description of embodiments of the invention taken in conjunction with the accompanying drawings, wherein:

Figure 1 is a partial perspective view of a patient, with an incision made along the greater trochanter to allow for implantation of a femoral implant of the present invention;

Figure 2 is a partial perspective view illustrating insertion of a guide plate in accordance with the present invention;

Figure 3 is a partial perspective view illustrating a guide tube/retractor in accordance with the present invention inserted through the incision aligned with the greater trochanter and engaged with the guide plate;

Figure 4 is an elevational view illustrating the use of an alignment device of the present invention to properly select the appropriate guide tube/retractor of the present invention;

Figure 5 is an elevational view illustrating the alignment guide of Figure 4 properly aligned from the greater trochanter along the femoral neck to the femoral head;

Figure 6 is a sectional view of a femur illustrating a plunge reamer utilized to begin making the femoral cavity of the present invention;

Figure 7 is a sectional view illustrating the use of a swivel reamer in accordance with the present invention to further form the femoral cavity;

Figure 8 is a sectional view illustrating further use of the swivel reamer depicted in Figure 7 to form the femoral cavity;

Figure 9 is a sectional view illustrating the use of a curved femoral head reamer to extend the femoral cavity into the femoral head;

Figure 10 is a sectional view illustrating the use of a curved femoral reamer to extend the femoral cavity into the intramedullary canal of the femur;

Figure 11 is a sectional view illustrating a femoral cavity formed in accordance with the present invention;

Figure 12 is a sectional view illustrating insertion of a femoral implant of the present invention into the femoral cavity illustrated in Figure 11;

Figure 13 is a sectional view illustrating extension of the bag of the femoral implant into the intramedullary canal;

Figure 14 is a sectional view illustrating extension of a lag screw through the lag screw tube and into the femoral head, as well as a pump and source of bag fill, e.g., bone cement, utilized to fill the bag of the femoral implant of the present invention;

Figure 15 is a perspective view of a guide plate in accordance with the present invention;

Figures 16, 17, and 18 are, respectively, top, side, and bottom elevational views thereof;

Figure 19 is a sectional view of an insertion member of the present invention with the guide plate illustrated in Figures 15-18 affixed thereto;

Figure 20 is a perspective view of an insertion member which is utilized to operably position a guide plate, e.g., the guide plate illustrated in Figures 15-18 atop the greater trochanter as illustrated in Figure 2;

Figure 21 is a partial elevational view illustrating deactuation of the latch utilized to temporarily fix the guide plate to the insertion member;

Figure 22 is a side elevational view of the insertion member illustrated, e.g., in Figure 20;

Figure 23 is a perspective view of a guide tube/retractor of the present invention;

Figure 24 is a radial elevational view thereof;

Figure 25 is a further radial elevational view thereof, rotated approximately 90 degrees with respect to the radial elevational view of Figure 24;

Figure 26 is a proximal axial view thereof;

Figure 27 is a distal axial view thereof;

Figure 28 is a radial elevational view of an angled guide tube/retractor of the present invention;

Figure 29 is a perspective view of an alignment device of the present invention;

Figure 30 is an elevational view thereof;

Figure 31 is a perspective view of a plunge reamer of the present invention;

Figure 32 is a distal axial view thereof;

Figure 33 is a partial sectional, elevational view thereof;

Figure 34 is a perspective view of a swivel reamer of the present invention;

Figure 35 is a proximal axial elevational view thereof;

Figure 36 is a sectional view taken along line 36-36 of Figure 38;

Figure 37 is a distal axial elevational view thereof;

Figure 38 is a partial sectional, radial elevational view of the swivel reamer of the present invention;

Figure 39 is a perspective view of a curved femoral head reamer of the present invention;

Figure 40 is a sectional view thereof;

Figure 41 is an elevational view of a femoral implant of the present invention;

Figure 42 is an exploded view of a lag screw of the present invention;

Figure 43 is a sectional view of the femoral implant of the present invention taken along line 43-43 of Figure 41;

Figure 44 is a perspective view of an alternative embodiment alignment device of the present invention;

Figure 45 is an elevational view thereof;

Figure 46 is a perspective view of a combination reamer in accordance with the present invention;

Figure 47 is a sectional view thereof illustrating actuation of the swivel/plunge reaming selector into the plunge reaming position;

Figure 48 is a sectional view thereof with the swivel/plunge reaming selector moved into position for swivel reaming;

Figure 49 is a partial sectional view of the combination reamer of the present invention;

Figure 50 is a perspective view of an alternative embodiment guide plate in accordance with the present invention;

Figures 51-54 are top, end, side, and bottom elevational views thereof, respectively;

Figure 55 is a sectional view thereof taken along line 55-55 of Figure 53;

Figure 56 is a perspective view of an alternative embodiment guide tube/retractor of the present invention;

Figure 57 is a radial elevational view thereof;

Figure 58 is a radial elevational view of an alternative embodiment angled guide tube/retractor of the present invention;

Figure 59 is a distal axial elevational view of the guide tube/retractor illustrated in Figure 57;

Figure 60 is a partial sectional view of the guide tube/retractor illustrated in Figure 57 taken along line 60-60 thereof;

Figure 61 is a perspective view of a fixation screw in accordance with an alternative embodiment of the present invention;

Figure 62 is a radial elevational view thereof;

Figure 63 is a distal axial view thereof;

Figure 64 is a proximal axial view thereof;

Figure 65 is a perspective view of a second alternative embodiment guide plate in accordance with the present invention;

Figure 66 is a top elevational view thereof;

Figure 67 is a sectional view thereof taken along line 67-67 of Figure 66;

Figure 68 is a bottom elevational view thereof;

Figure 69 is a perspective view of a second alternative embodiment guide tube/retractor in accordance with the present invention;

Figure 70 is a radial elevational view thereof;

Figure 71 is an exploded view of a flexible reamer guide in accordance with the present invention;

Figure 72 is a sectional view thereof;

Figure 73 is a sectional view illustrating the flexible reamer guide of Figures 71 and 72 operably positioned within a patient's femur to guide a flexible reamer into the femoral head;

Figure 74 is a sectional view illustrating a flexible reamer positioned over a flexible reamer guide wire for reaming into the femoral head;

Figure 75 is a perspective view of a flexible reamer in accordance with the present invention;

Figure 76 is a sectional view thereof;

Figure 77 is an exploded view of a flexible reamer guide wire bender in accordance with the present invention;

Figure 78 is an elevational view thereof;

Figure 79 is a sectional view thereof;

Figure 80 is an axial elevational view of the distal end of a fixation screw placement instrument in accordance with the present invention;

Figure 81 is a perspective view of the fixation screw placement instrument partially illustrated in Figure 80;

Figure 82 is a perspective view of a straight reamer utilized to prepare the greater trochanter to receive the fixation screw illustrated in Figure 61-64;

Figure 83 is a perspective view of an alternative embodiment insertion member for inserting a guide plate of the present invention;

Figure 84 is a partial sectional view thereof illustrating the release bars thereof actuated to effect release of the guide plate from locking engagement with the insertion member;

Figure 85 is a partial sectional view illustrating the release bars of the insertion member illustrated in Figure 83 positioned whereby the guide plate can be temporarily fixed to the insertion member;

Figure 86 is an elevational view of the insertion member illustrated in Figure 83;

Figure 87 is a perspective view of a spring lock release instrument in accordance with the present invention;

Figure 88 is a partial sectional view of the distal end thereof, illustrating the release pins in an unactuated position;

Figure 89 is a sectional view of the spring lock release instrument of Figure 87 actuated to force release pins 346 to protrude therefrom;

Figure 90 is an elevational view of an alternative embodiment femoral implant of the present invention;

Figure 91 is a sectional view of an alternative embodiment lag screw of the present invention, illustrating insertion of an actuating device for actuating the lag screw head;

Figure 92 is a partial sectional view of a further alternative embodiment lag screw of the present invention;

Figure 93 is a partial elevational view of a femur illustrating insertion of a guide wire to guide reaming from the greater trochanter into the femoral head;

Figure 94 is a partial elevational view of a femur illustrating use of a flexible reamer having two reaming diameters to ream a passage from the greater trochanter into the femoral head;

Figure 95 is a partial radial elevational view of a flex up reamer for reaming a passage from the greater trochanter into the femoral head;

Figure 96 is a distal axial elevational view thereof;

Figure 97 is a radial elevational view of a telescoping reamer of the present invention illustrating extension of a reaming head therefrom;

Figure 98 is a radial elevational view of the telescoping reamer of Figure 97 shown in its retracted position;

Figure 99 is an exploded view of the telescoping reamer of Figures 97 and 98;

Figure 100 is a perspective view of a swivel/down reamer assembly shown in unactuated position;

Figure 101 is a perspective view thereof shown in actuated position;

Figure 102 is an exploded view of the swivel/down reamer assembly illustrated in Figures 100 and 101;

Figure 103 is a partial elevational view illustrating use of the swivel/down reamer assembly depicted in Figures 100-102 to extend the femoral cavity into the intramedullary canal;

Figure 104 is a sectional view of the tool housing of the swivel/down reamer assembly depicted in Figures 100-102;

Figure 105 is a radial elevational view of a flexible guide shaft of the swivel/down reamer assembly depicted in Figures 100-102;

Figure 106 is an axial elevational view thereof;

Figure 107 is a perspective view of a unitube retractor of the present invention with the ball detent retaining mechanism thereof illustrated in position to retain an instrument within the unitube retractor;

Figure 108 is a perspective view of the unitube retractor of Figure 107 illustrating the ball detent retaining mechanism actuated to allow for release of an instrument positioned within the unitube retractor;

Figure 109 is an exploded perspective view of the unitube retractor illustrated in Figures 107 and 108;

Figure 110 is a sectional view of a plunger forming a part of the ball detent retaining mechanism depicted with the unitube retractor of Figures 107-109;

Figure 111 is an exploded perspective view of an alternative embodiment unitube retractor in accordance with the present invention;

Figure 112 is a sectional view of the lock ring of the unitube retractor depicted in Figure 111;

Figure 113 is a radial elevational view of the unitube retractor illustrated in Figure 111 shown in unactuated position;

Figure 114 is a radial elevational view illustrating the unitube retractor of Figures 111 and 113 in actuated position, with the fingers of the lock ring thereof radially expanded to lock the unitube retractor to the femur through the access formed therein; and

Figure 115 is a partial radial elevational view thereof.

Corresponding reference characters indicate corresponding parts throughout the several views. Although the drawings represent embodiments of the present invention, the drawings are not necessarily to scale and certain features may be exaggerated to better illustrate and explain the present invention. The exemplifications set out herein illustrate embodiments of the invention, and such exemplifications are not to be construed as limiting the scope of the invention in any manner.

Throughout this document, "proximal" and "distal" are used to refer to opposite ends of instruments described herein. When referring to the opposite ends of instruments, "proximal" and "distal" are used with reference to a user of the instrument. For example, the end of the instrument nearest to the user during use thereof is described as the proximal end, while the end of the instrument farthest from the user during use thereof is described as the distal end of the instrument.

### DETAILED DESCRIPTION OF THE INVENTION

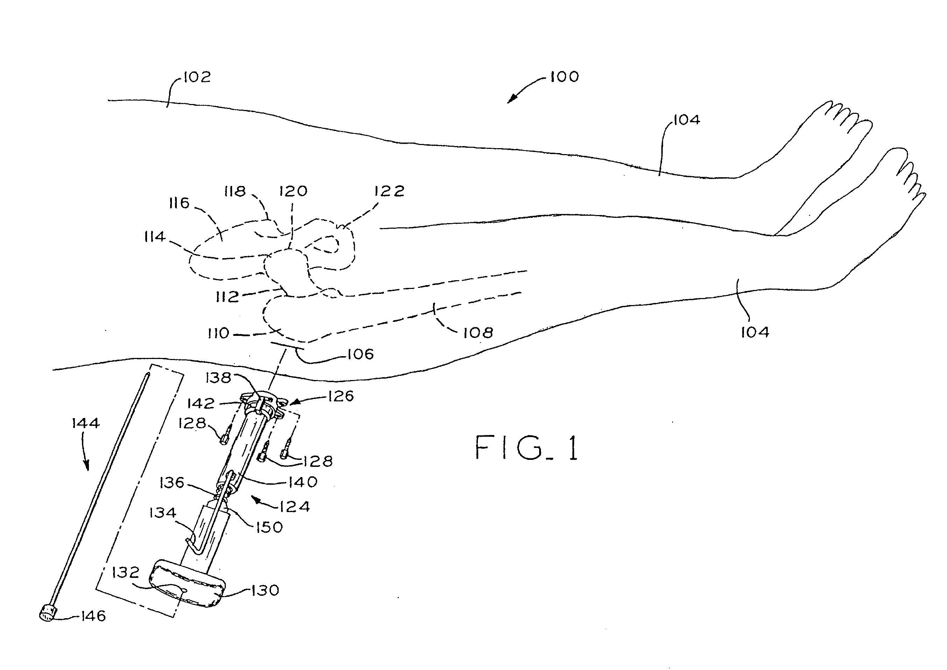

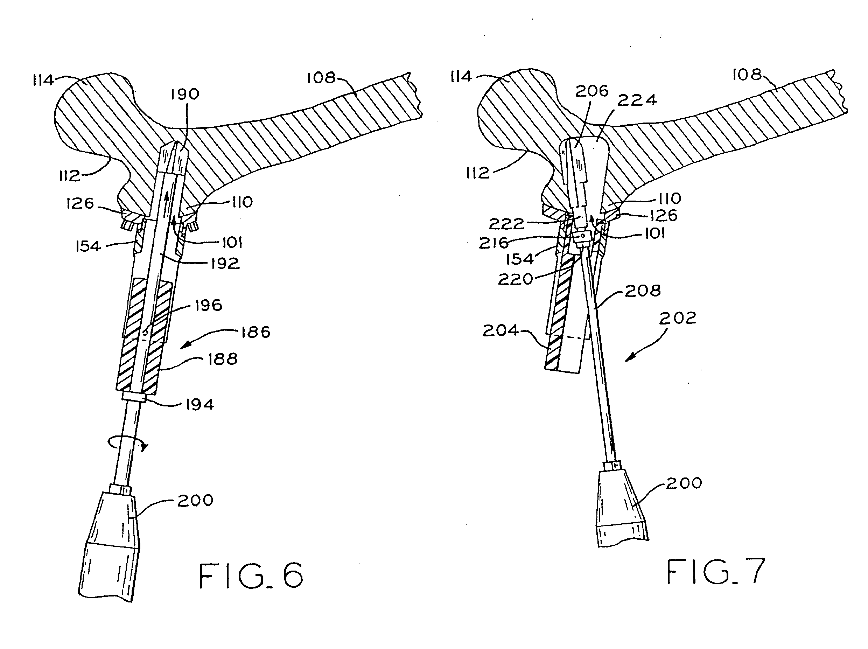

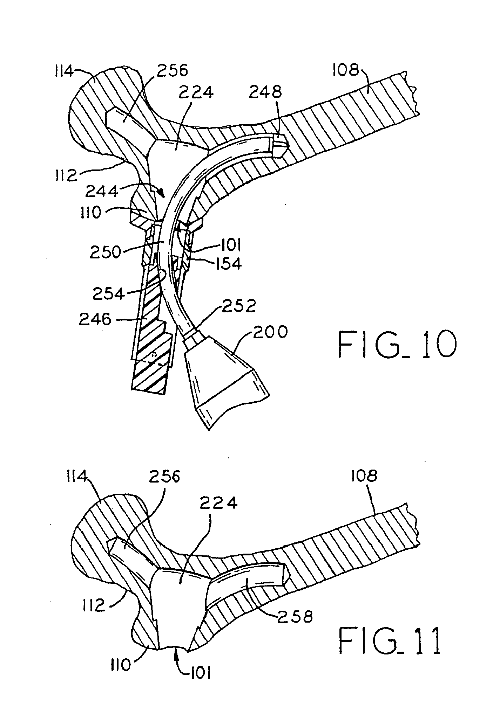

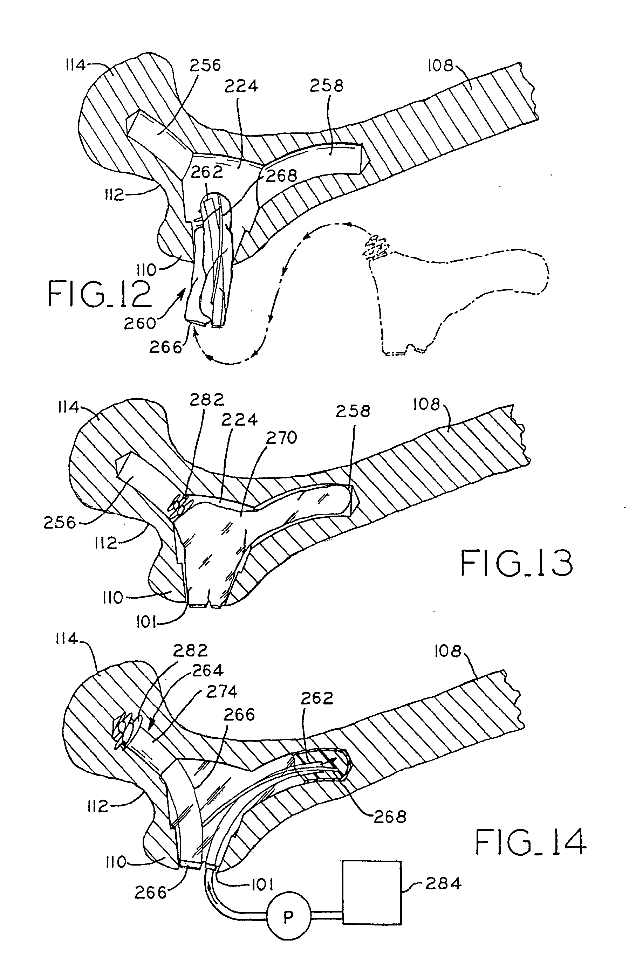

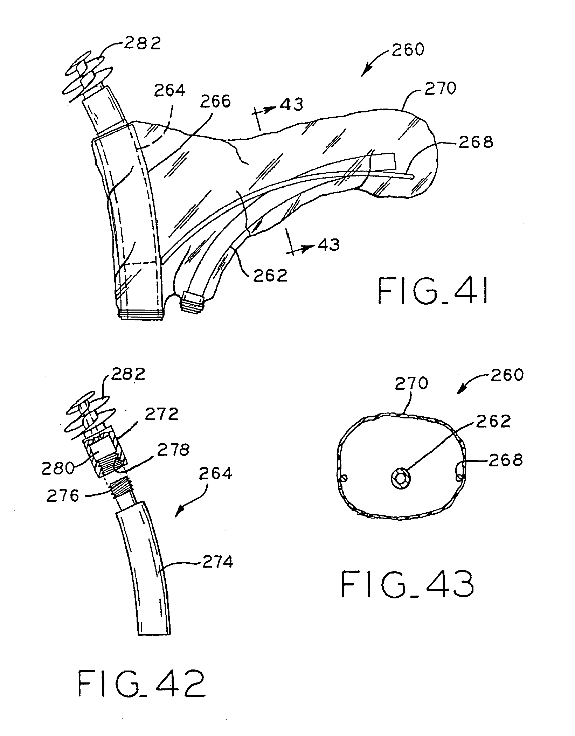

Implant 260 illustrated in Figure 41 is utilized to reduce a femoral fracture utilizing a method of implantation which does not require incision of the quadriceps. As illustrated in Figure 1, incision 106 is aligned with greater trochanter 110, with femur 108 being prepared to receive implant 260 through incision 106. As described above, greater trochanter 110 is not covered with muscle and therefore, incision 106 can be developed to expose greater trochanter 110 without requiring the incision of muscle. Incision 106 measures about 2.5 centimeters (1 inch). Figures 6-10 illustrate use of various novel reamers of the present invention to form femoral cavity 224 (Figure 11). Various instruments described below may be utilized in lieu of or in conjunction with the instruments illustrated in Figures 6-10. As illustrated in Figure 12, implant 260 (further illustrated in Figures 41-43) is inserted into femoral cavity 224 via access 101 (Figures 13 and 14) formed through greater trochanter 110. As illustrated in Figure 13, lag screw 264 is advanced into femoral head 114 until lag screw threads 282 firmly engage femoral head 114 and lag screw 264 has achieved the position illustrated in Figure 14. Bag 270 is thereafter filled with material, e.g., bone cement to fill femoral cavity 224 and provide intramedullary fixation of implant 260 and stabilization of lag screw 264. In this way, a femoral fracture including, e.g., an intertrochanteric fracture can be reduced. Generally, this document will refer to a femoral fracture and, specifically, to an intertrochanteric fracture. However, the method and apparatus of the present invention is adaptable to various bone fractures including, e.g., supracondylar fractures of the femur.

Figure 1 generally illustrates patient 100 including torso 102, and legs 104. Figure 1 further illustrates the general bone structures comprising the hip joint including, pubis 122, anterior superior iliac spine 118, ilium 116, acetabulum 120, and femur 108. As illustrated in Figure 1, femur 108 includes, e.g., greater trochanter 110, femoral neck 112, and femoral head 114. As described above, incision 106 is aligned with greater trochanter 110. Because greater trochanter 110 is not covered with muscle, incision 106 can be made and the wound developed through the skin and fascia to expose greater trochanter 110 without incising muscle, including, e.g., the quadriceps.

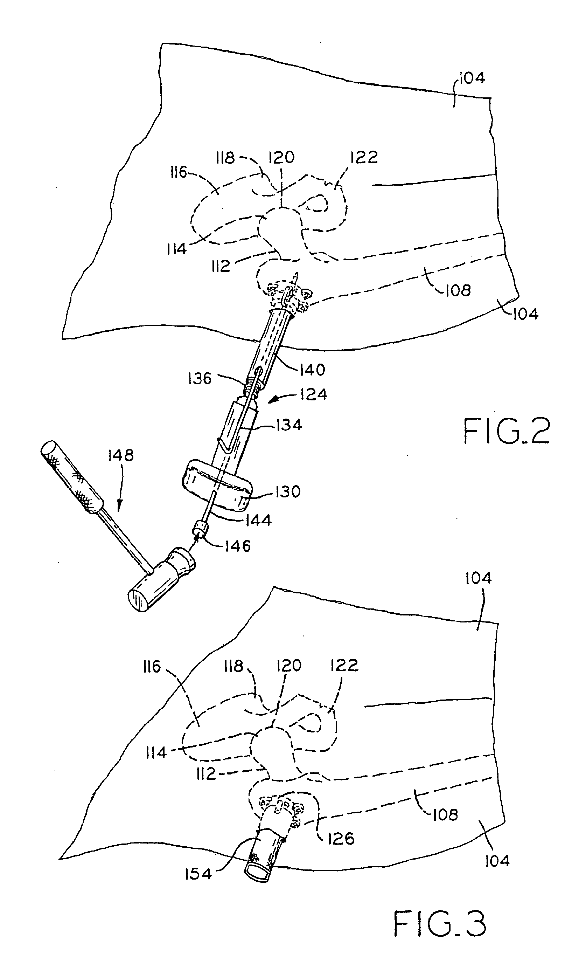

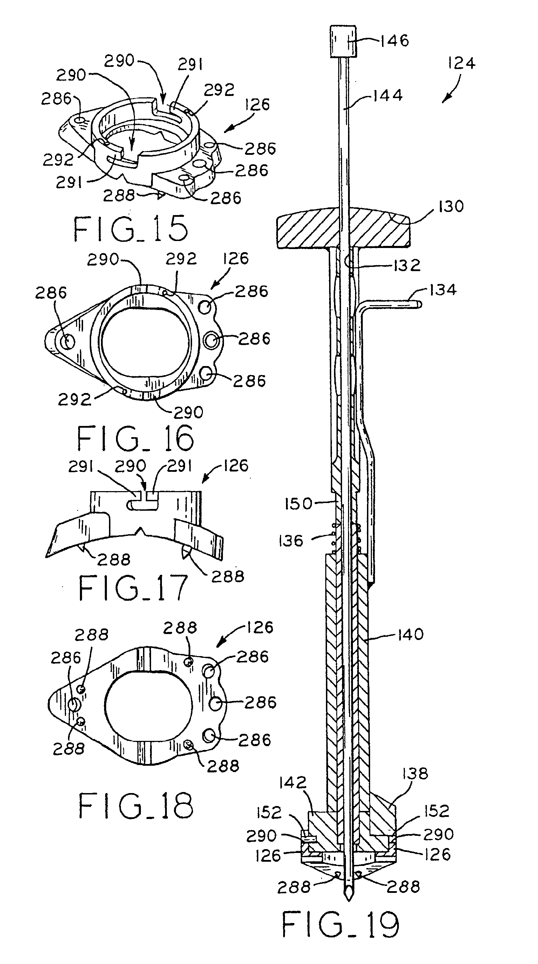

In one embodiment of the present invention, cannulated insertion member 124 is utilized to insert guide plate 126 through incision 106 to be placed atop and secured to greater trochanter 110 as illustrated, e.g., in Figure 2. After guide plate 126 traverses incision 106 and is placed atop greater trochanter 110, stabilization nail 144 is positioned through elongate aperture 132 (Figure 19) of insertion member 124 and impaction instrument 148 (Figure 2) is utilized to strike impaction surface 146 to drive stabilization nail 144 into femur 108 to provide initial stability to guide plate 126 prior to utilizing screws 128 (Figure 1) to fix guide plate 126 to greater trochanter 110. In one exemplary embodiment, the surgeon implanting guide plate 126 will utilize a fluoroscope to verify proper placement of guide plate 126 atop greater trochanter 110. In alternative embodiments, the surgeon implanting guide plate 126 will utilize tactile feedback either alone or in conjunction with a fluoroscope image to determine proper placement of guide plate 126 atop greater trochanter 110. After guide plate 126 is properly positioned atop greater trochanter 110, screws 128 are driven through corresponding screw apertures 286 (Figure 15) in guide plate 126 and into femur 108 to secure guide plate 126 to femur 108. Screw apertures 286 are, in one exemplary embodiment, formed in guide plate 126 to allow for oblique insertion of screws 128 relative to guide plate 126.

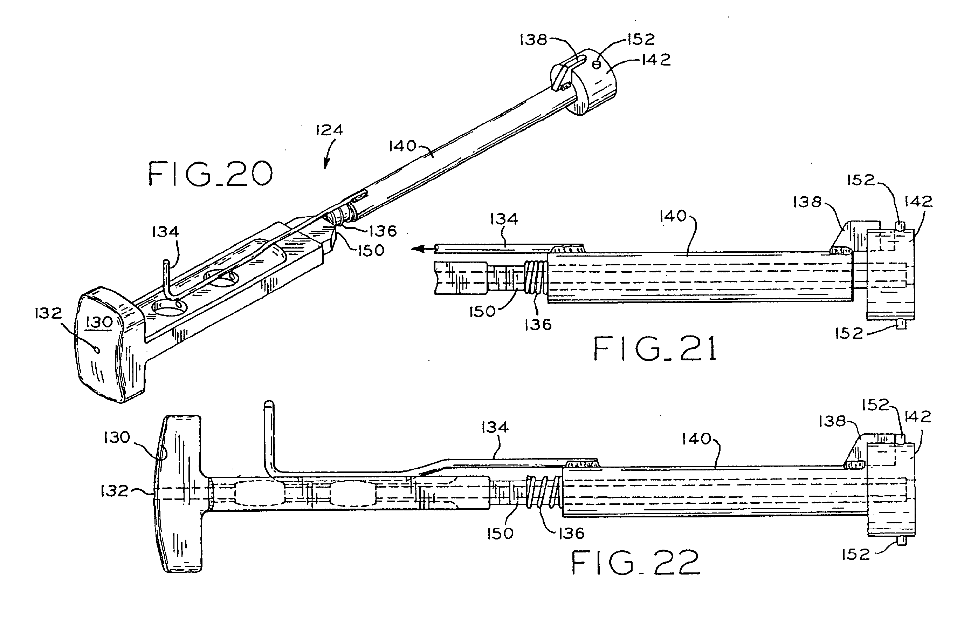

Insertion member 124 is illustrated in detail in Figures 19-22. As illustrated, insertion member 124 includes elongate aperture 132 accommodating stabilization nail 144 as described hereinabove. Insertion member 124 includes tubular latch connector 140 positioned about the distal end thereof. Intermediate the main body of insertion member 124 and tubular latch connector 140 is positioned spring 136. Spring 136 acts against spring stop 150 to bias tubular latch connector into the position illustrated in Figure 22. Release member 134 is connected to tubular latch connector 140 and is operable to facilitate movement of tubular latch connector 140 against the biasing force of spring 136 into the position illustrated in Figure 21. Insertion member 124 includes distal end 142 for engaging guide plate 126. Distal end 142 includes bosses 152 extending therefrom.

Guide plate 126 is temporarily affixed to insertion member 124 as described below. Bosses 152 of insertion member 124 enter attachment channels 290 of guide plate 126 (see, e.g., Figures 15 and 17). Concurrently, latch 138, connected to tubular latch connector 140, acts against the proximal surface of guide plate 126 to force tubular latch connector 140 against the biasing force of spring 136 and into the position illustrated in Figure 21. Distal end 142 of insertion member 124 is then rotated until bosses 152 are positioned under lips 291 formed by attachment channels 290 and latch 138 can be positioned within one of attachment channels 290 and returned to its naturally biased position as illustrated in Figures 19 and 22. When guide plate 126 is attached to insertion member 124, one of bosses 152 and latch 138 abut opposing radial extremes of one attachment channel 290 to prevent relative rotation of guide plate 126 and insertion number 124. Moreover, when guide plate 126 is attached to insertion member 124, bosses 152 cooperate with lips 291 formed by attachment channels 290 to prevent relative axial displacement of guide plate 126 and insertion member 124. In this way, guide plate 126 is secured to insertion member 124 to facilitate positioning guide plate 126 atop greater trochanter 110 as described hereinabove.

After guide plate 126 is secured to greater trochanter 110, release member 134 may be actuated to position latch 138 in the position illustrated in Figure 21 to allow for rotation of distal end 142 of insertion member 124 relative to guide plate 126. When latch 138 is positioned as illustrated in Figure 21, it is no longer contained within attachment channel 290 and therefore allows relative rotation between guide plate 126 and insertion member 124. Distal end 142 of insertion member 124 is rotated to reposition bosses 152 out of axial alignment with lips 291 for removal from attachment channels 290. Insertion member 124 is thereafter removed from engagement with guide plate 126 and removed through incision 106.

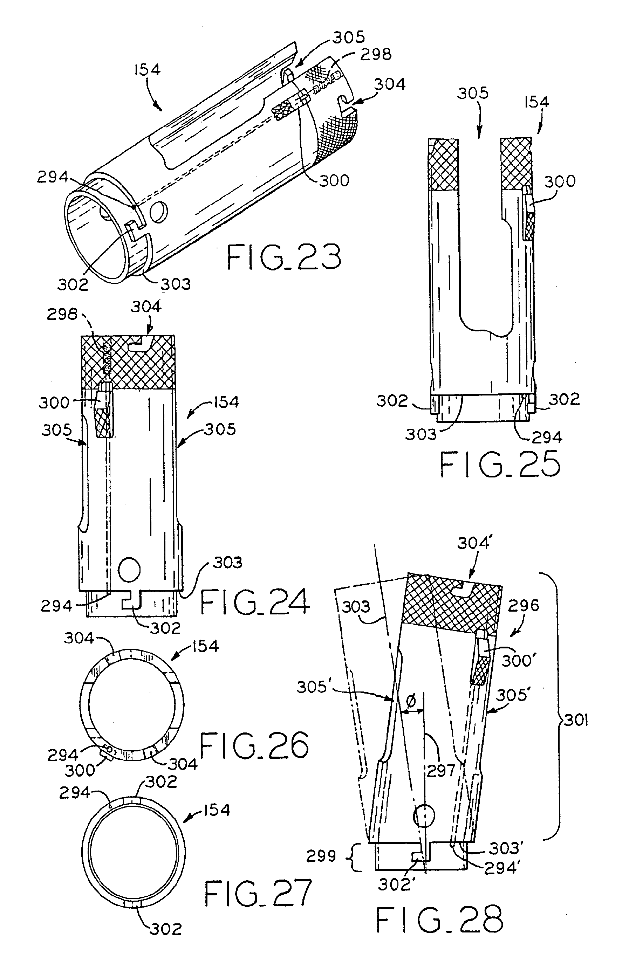

After securement of guide plate 126 atop greater trochanter 110, guide tube/retractor 154 (Figures 23-27) is inserted through incision 106 and releasably fixed to guide plate 126 as illustrated in Figure 3. Guide tube/retractor 154 is illustrated in detail in Figures 23-27, and guide plate 126 is illustrated in detail in Figures 15-18. With reference to Figures 23-27 and 15-18, the cooperating apparatus of guide tube/retractor 154 and guide plate 126 allowing for selective locking of guide tube/retractor 154 to guide plate 126 will now be described. Fixation of guide tube/retractor 154 to guide plate 126 is effected by first positioning attachment protrusions 302 of straight guide tube/retractor 154 into attachment channels 290 of guide plate 126. Guide tube/retractor 154 is then rotated clockwise to position the radially extending portion of attachment protrusions 302 under lips 291 formed by attachment channels 290 of guide plate 126. Once rotated into this position, spring biased locking pin 294 of guide tube/retractor 154 is positioned within lock detent 292 of guide plate 126 to prevent relative rotation of guide plate 126 and guide tube/retractor 154 and lock guide tube/retractor 154 to guide plate 126.

As illustrated in Figures 23 and 24, spring biased locking pin 294 extends substantially axially along guide tube/retractor 154 and is operably connected to actuation member 300 to provide for manual actuation of locking pin 294. Spring 298 is operatively associated with spring biased locking pin 294 and the interior of the cylindrical wall forming guide tube/retractor 154 to bias locking pin 294 into the position illustrated in Figure 24. When distal shoulder 303 of guide tube/retractor 154 is initially positioned atop the proximal end of guide plate 126, with attachment protrusions 302 entering attachment channels 290, locking pin 294 is moved against the biasing force of spring 298 until guide tube/retractor 154 is rotated as described hereinabove to align locking pin 294 with detent 292 and lock guide tube/retractor 154 to guide plate 126.

While the engagement of a guide tube/retractor of the present invention with guide plate 126 has been described with respect to straight guide tube/retractor 154, angled guide tube/retractor 296 (illustrated in Figure 28 and described below) is locked to guide plate 126 in the same manner utilizing the same structure as described above with respect to straight guide tube/retractor 154. The shared components of straight guide tube/retractor 154 and angled guide tube/retractor 296 are denoted with primed reference numerals. The mechanism for locking a guide tube/retractor of the present invention to guide plate 126 allows for locking of a guide tube/retractor to guide plate 126 in one of two positions separated by 180 degrees. This allows for angled guide tube/retractor 296 to provide for realignment in two directions as further described hereinbelow.

Guide tube/retractor 154 serves the dual purpose of maintaining an access from incision 106 to greater trochanter 110 and guiding various instruments utilized to prepare femoral cavity 224 (Figure 11). Generally, either a straight or an angled guide tube/retractor will be utilized. Figures 24 and 28 respectively illustrate straight guide tube/retractor 154 and angled guide tube/retractor 296. As illustrated, e.g., in Figure 28, angled guide tube/retractor 296 includes distal end 299 and retractor body 301. Longitudinal axis 297 of distal end 299 of angled guide tube/retractor 296 forms an angle Ø of about 10° with longitudinal axis 303 of retractor body 301. In this way, angled guide tube/retractor 296 allows for a 10° realignment with respect to straight guide tube/retractor 154. A surgeon can choose either straight guide tube/retractor 154 or angled guide tube/retractor 296 based upon the geometry of femur 108 into which implant 260 (Figure 41) will be placed. In accordance with the present invention, an alignment device is provided to facilitate choice of straight guide tube/retractor 154 or angled guide tube/retractor 296 as well as the orientation of angled guide tube/retractor 296 as further described hereinbelow.

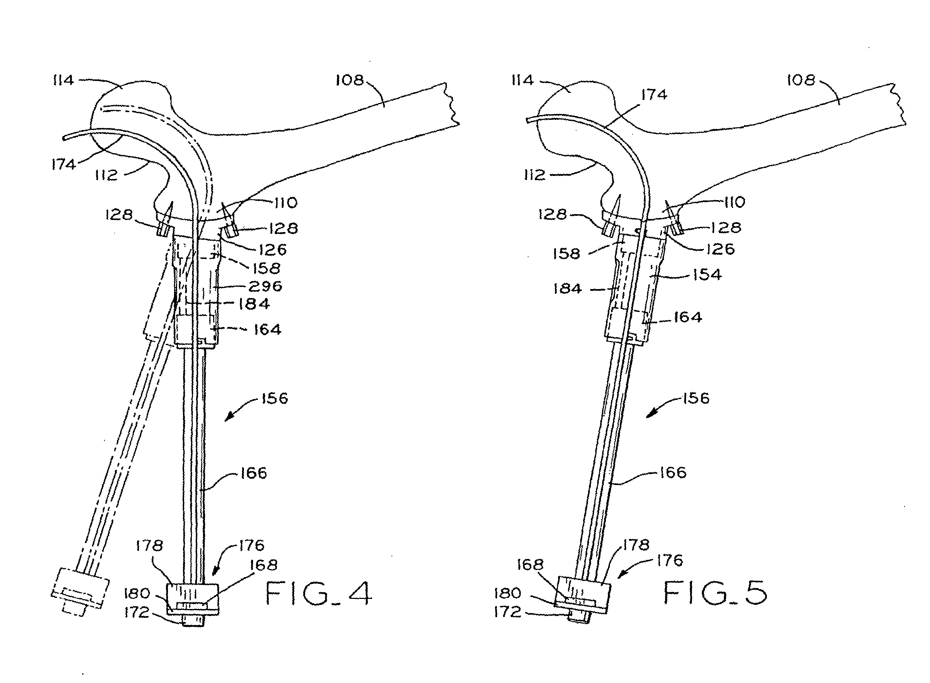

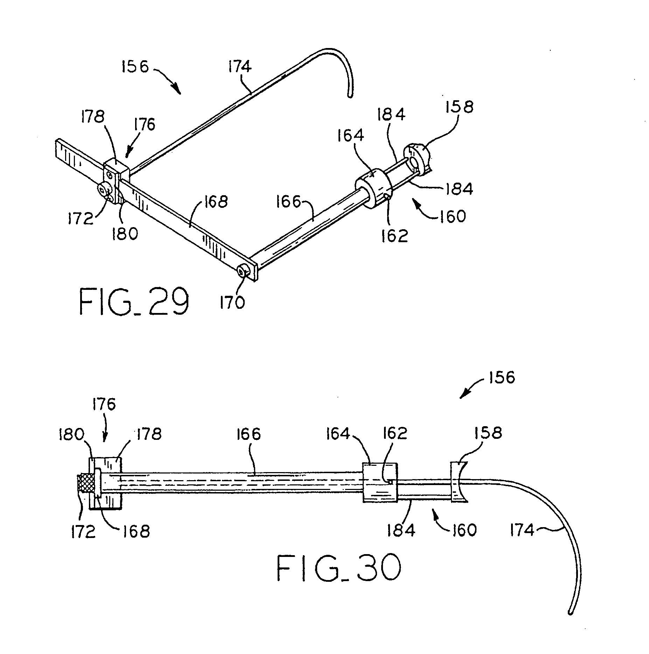

Figures 4 and 5 illustrate use of alignment device 156 to choose either straight guide tube/retractor 154 or angled guide tube/retractor 296. Alignment device 156 is illustrated in detail in Figures 29 and 30 and includes extension 166 connected to transverse bar 168, with alignment arm 174 slidably attached thereto. As illustrated in Figure 29, extension 166 is connected to insertion member 160 at a distal end thereof. Insertion member 160 is sized for insertion into either straight guide tube/retractor 154 or angled guide tube/retractor 296 as illustrated in Figures 4 and 5.

As illustrated in Figures 29 and 30, insertion portion 160 of alignment device 156 includes distal end 158 connected via connecting rods 184 to positioning cylinder 164. Positioning cylinder 164 includes a pair of opposing bosses 162, only one of which is depicted in Figures 29 and 30. Distal end 158 and positioning cylinder 164 have external geometries sized to cooperate with the hollow interior of the guide tube/retractors of the present invention to provide a stationary base for alignment device 156, as illustrated in Figures 4 and 5. Insertion portion 160 of alignment device 156 as illustrated in Figures 29 and 30 comprises merely one exemplary design for an insertion portion of alignment device 156 operable to stabilize alignment device 156 with the guide tube/retractors of the present invention. Generally, insertion portion 160 will include a portion thereof having an exterior geometry sized to cooperate with the interior of the guide tube/retractors of the present invention to provide a stationary base for alignment device 156. In an alternative embodiment, the insertion portion of alignment device 156 depicted in Figures 29 and 30 comprises a solid insertion member having a consistent cross sectional area along its length. In this embodiment, the exterior of the solid insertion member will cooperate with the interior of the guide tube/retractors of the present invention to provide a stable connection of alignment device 156 with a guide tube/retractor in accordance with the present invention.

Alignment device 156 includes transverse bar 168 fixed to extension 166 via screw 170. Positioning cylinder 164 and extension 166 provide a stable base for transverse bar 168. As illustrated in Figures 29 and 30, alignment arm 174 is slidably connected to transverse bar 168 via slidable attachment member 176. Slidable attachment member 176 includes attachment block 178 having a cutout therein accommodating transverse bar 168. Top plate 180 is mounted atop attachment block 178, with set screw 172 threaded therein. Set screw 172 traverses top plate 180 to selectively engage transverse bar 168 and lock alignment arm 174 in position along transverse bar 168.

As illustrated in Figures 4 and 5, alignment device 156 is utilized to facilitate selection of the appropriate guide tube/retractor. Figure 5 illustrates alignment device 156 operably positioned within straight guide tube/retractor 154, which is locked to guide plate 126. In use, bosses 162 on positioning cylinder 164 are positioned within attachment channels 290 of guide plate 156 and positioning cylinder 164 is rotated until bosses 162 contact the terminal ends of channels 290 and are positioned under lips 291. After positioning alignment device 156 within guide tube/retractor 154, slidable attachment member 176 may be adjusted to accommodate the physiological characteristics of the patient and place alignment arm 174 adjacent the patient's skin. Alignment arm 174 of alignment device 156 includes a curved distal end having a curvature based on statistical data which follows a path from the central portion of greater trochanter 110, along the central axis of femoral neck 112, to the central region of femoral head 114. Figure 5 illustrates an arrangement with the distal end of alignment arm 174 following the aforementioned path on femur 108. In the environment illustrated in Figure 5, straight guide tube/retractor 154 is the appropriate guide tube/retractor to be utilized to effect the method of the present invention. In some cases, the distal end of alignment arm 174 will not coincide with the aforementioned path on the femur in question due to, e.g., the specific geometry of the bone in question. In this case, angled guide tube/retractor 296 may be utilized in an attempt to provide the appropriate alignment with the femur in question.

Figure 4 illustrates alignment device 156 utilized with angled guide tube/retractor 296 on femur 108. As described above, femur 108, illustrated, e.g., in Figures 4 and 5 has a geometry accommodating the use of straight guide tube/retractor 154. With this in mind, Figure 4 is useful in illustrating a situation in which the distal end of alignment arm 174 does not follow a path from the central portion of greater trochanter 110, along the central axis of femoral neck 112 to the central region of femoral head 114 and, therefore, use of the attached guide tube/retractor, i.e., angled guide tube/retractor 296 is contraindicated. Comparison of the distal end of alignment ann 174 to the aforementioned path from the central portion of the greater trochanter, along the central axis of the femoral neck to the central portion of the femoral head will be effected during surgery with the use of a fluoroscope.

Generally, straight guide tube/retractor 154 will first be locked to guide plate 126, and alignment device 156 will be operably positioned therein. A fluoroscope will then be utilized to compare the distal end of alignment arm 174 with the path from the central portion of the greater trochanter, along the central axis of the femoral neck to the central portion of the femoral head. If the distal end of alignment arm 174 does not follow the aforementioned path from the central portion of the greater trochanter to the central portion of the femoral head, then alignment device 156 and straight guide tube/retractor 154 will be removed and angled guide tube retractor 296 will be locked to guide plate 126. The angle Ø of about 10° formed between longitudinal axis 297 of distal end 299 of angled guide tube/retractor 296 and longitudinal axis 303 of retractor body 301 allows for an approximately 10 degree realignment on either side of the longitudinal axis of straight guide tube/retractor 154 in a plane substantially containing the central axis of femur 108. The inventors of the current invention have found that this 10 degree realignment in either direction typically accounts for the various bone geometries encountered. However, the inventors of the present invention further contemplate provision of additional angled guide tubes/retractors having an angle Ø as described hereinabove of other than 10 degrees. For example, 0 could measure 5°, 10°, or 15° to provide for increased versatility in performing the method of reducing a femoral fracture in accordance with the present invention.

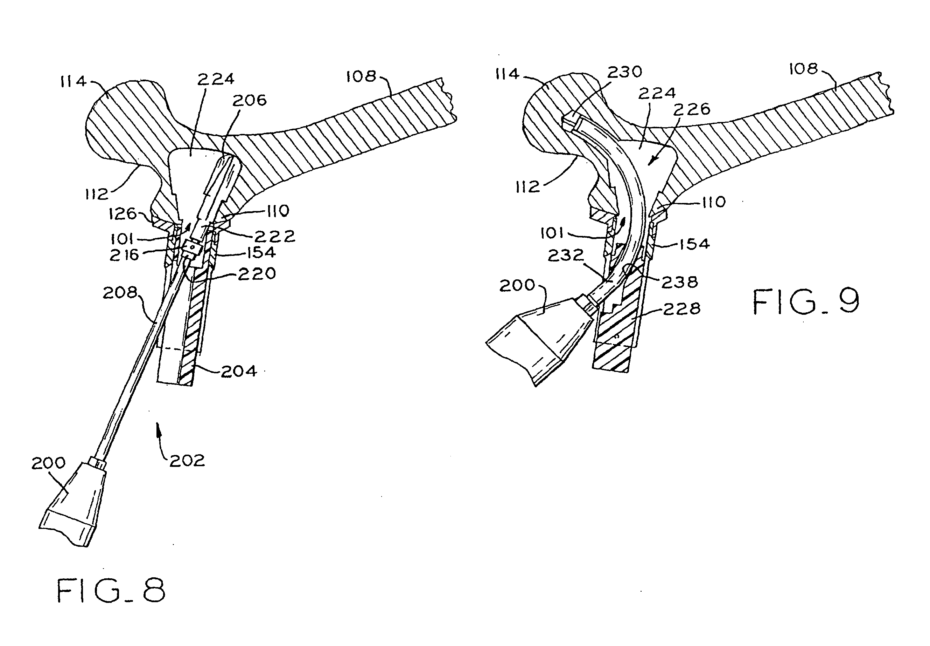

Once the appropriate guide tube/retractor is chosen and attached to guide plate 126, cavity 224 (Figure 11) can be formed in femur 108. As illustrated in Figure 6, straight reamer 186 is first positioned within guide tube/retractor 154 and utilized to create access 101 in greater trochanter 110. In one exemplary embodiment, access 101 has a 1.9 centimeter (0.75 inch) diameter. After creating access 101 in greater trochanter 110, straight reamer 186 is removed from guide tube/retractor 154 and replaced with swivel reamer 202 as illustrated, e.g., in Figure 7. As illustrated in Figure 7, swivel reamer 202 is rotatable about pivot 216 and, in the configuration illustrated in Figure 7, allows for the extension of femoral cavity 224 toward femoral head 114. After femoral cavity 224 is extended as illustrated in Figure 7, swivel reamer 202 is repositioned to allow for extension of femoral cavity 224 toward the shaft of femur 108 as illustrated in Figure 8. Swivel reamer 202 is then removed in favor of curved femoral head reamer 226. As illustrated in Figure 9, curved femoral head reamer 226 is advanced through access 101 into femoral head 114, thus expanding femoral cavity 224 into femoral head 114. Curved femoral head reamer 226 is thereafter removed from guide tube/retractor 154 and replaced with curved femoral shaft reamer 244, as illustrated in Figure 10. Curved femoral shaft reamer 244 is positioned through access 101 into the intramedullary canal of femur 108, as illustrated in Figure 7, to extend femoral cavity 224 into the femoral shaft. The reaming process illustrated in Figures 6-10 produces femoral cavity 224 as illustrated, e.g., in Figure 11.

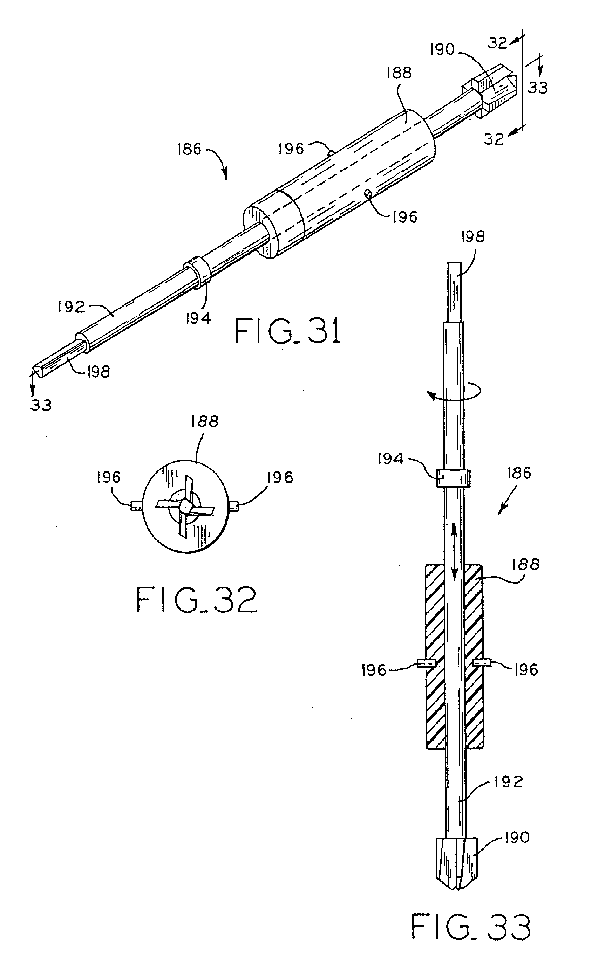

Straight reamer 186 is illustrated in detail in Figures 31-33. As illustrated in Figures 31-33, straight reamer 186 includes straight reamer guide tube 188 surrounding straight reamer shaft 192. Straight reamer guide tube 188 is positioned intermediate straight reamer head 190 and flange 194 and is operable to move along reamer shaft 192 therebetween. Straight reamer guide tube 188 as an exterior geometry cooperating with the internal geometry of straight guide tube/retractor 154 and/or angled guide tube/retractor 296 to provide a solid base for reaming femur 108 as illustrated in Figure 6. Straight reamer 186 further includes proximal end 198 adapted to be received in chuck 200 (Figure 6) of any of the well known rotation devices utilized to impart rotational motion to various medical instruments including, e.g., reamers. Straight reamer guide tube 188 includes opposing bosses 196 protruding from the exterior surface thereof. Bosses 196 are engagable in boss channels 304 formed in the proximal end of the guide tube/retractors of the present invention (see, e.g., Figures 23, 24, and 28).

In use, straight reamer guide tube 188 is positioned within a guide tube/retractor of the present invention, with bosses 196 entering boss channels 304 formed in a proximal end thereof. Guide tube 188 is then rotated until bosses 196 are positioned beneath the lip formed by the proximal end of straight guide tube/retractor of the present invention covering the radially extending portions of boss channels 304. In this position, guide tube 188 cannot readily be axially displaced relative to the guide tube/retractor into which it is inserted. Proximal end 198 of straight reamer 186 is actuated to provide rotational movement of reamer head 190 to form access 101 in femur 108. After achieving a predetermined reamer depth, flange 194 contacts the proximal end of guide tube 188 to limit axial displacement of reamer head 190. In one exemplary embodiment, straight reamer 186 is configured to provide a reaming depth of 1.9 centimeters (.75 inches) into femur 108.

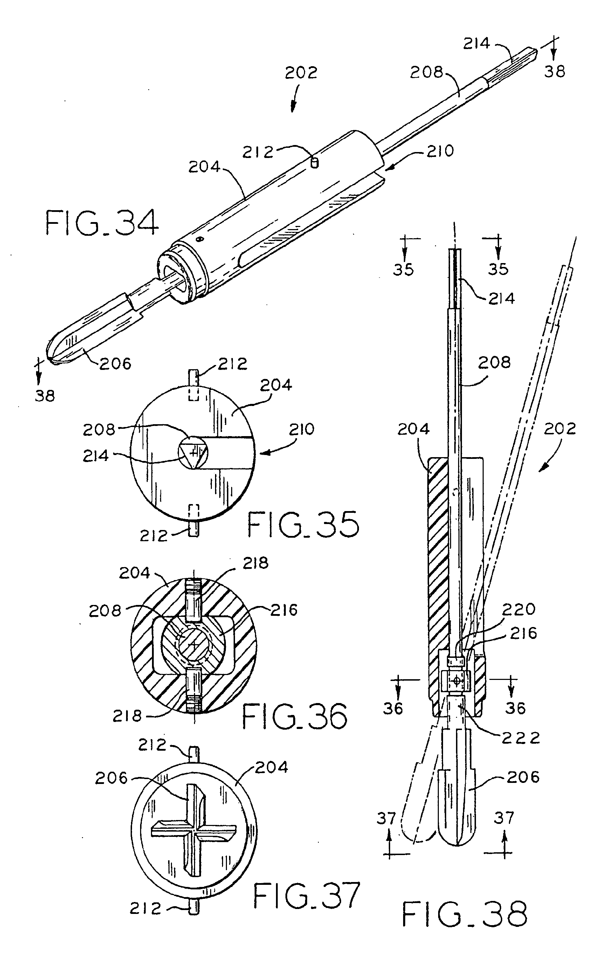

Swivel reamer 202 is illustrated in detail in Figures 34-38. As illustrated in Figures 34-38, swivel reamer 202 includes swivel reamer guide tube 204 having opposing bosses 212 protruding therefrom. Swivel reamer guide tube 204 includes cutout 210 operable to allow reamer shaft 208 to pivot about swivel reamer pivot 216 as further described hereinbelow and as illustrated in Figure 38. Similar to straight reamer 186, swivel reamer 202 includes proximal end 214 operable to connect swivel reamer 202 to chuck 200 (Figure 7). Bosses 212 are utilized to connect swivel reamer 202 to a guide tube/retractor of the present invention in the same manner as bosses 196 of straight reamer 186.

As illustrated in Figure 36, swivel reamer pivot 216 is pivotally connected to swivel reamer guide tube 204 via pivot pins 218. As illustrated in Figure 38, swivel reamer pivot 216 is positioned about reamer shaft 218 and abuts enlarged portion 222 of swivel reamer shaft 208 and flange 220 on opposing axial ends thereof to prevent axial displacement of swivel reamer head 206. As illustrated in Figures 7 and 8 and described hereinabove, the orientation of swivel reamer 202 is changed 180 degrees to accommodate swivel reaming toward femoral head 114 as illustrated in Figure 7 as well as swivel reaming toward the femoral shaft as illustrated in Figure 8. As illustrated, e.g., in Figures 23-25 and 28, the guide tube/retractors of the present invention includes opposing cut-outs 305 to accommodate swivel reaming toward femoral head 114 as illustrated in Figure 7 as well as swivel reaming toward the femoral shaft as illustrated in Figure 8, without repositioning the guide tube/retractor.

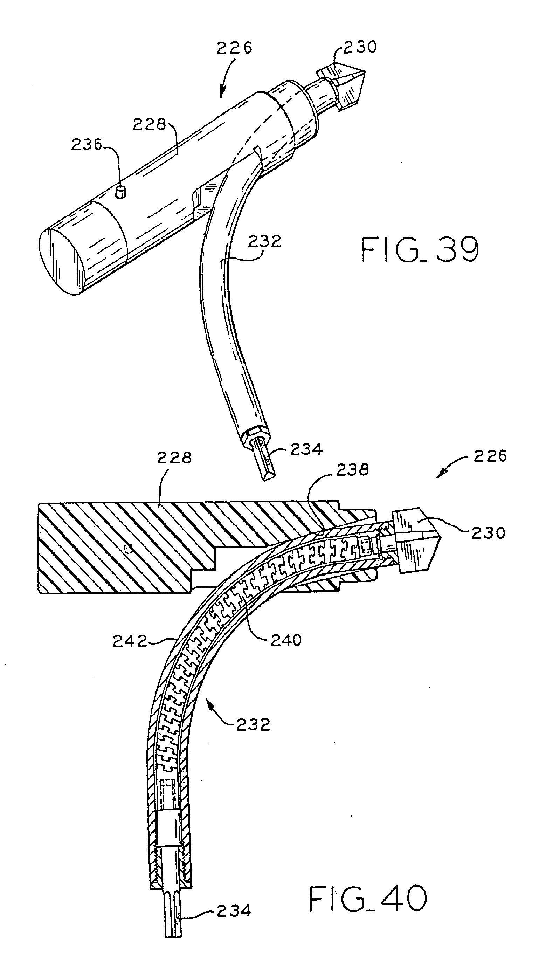

Curved femoral head reamer 226 is illustrated in detail in Figures 39 and 40. As illustrated in Figures 39 and 40, curved femoral head reamer 226 includes guide tube 228 having bosses 236 protruding therefrom. Bosses 236 are utilized to position curved femoral head reamer 226 within a guide tube/retractor of the present invention as described above with respect to straight reamer 186 and swivel reamer 202. Curved femoral head reamer 226 includes curved reamer shaft 232 having reamer head 230 operably connected to a distal end thereof. Proximal end 234 of curved reamer shaft 232 is operable to connect curved reamer 226 to chuck 200 of an actuation device as illustrated in Figure 9. As illustrated in Figure 40, curved reamer shaft 232 comprises a hollow shaft formed by outer tube 242. Flexible driveshaft 240 is positioned within outer tube 242 and allows for transmission of rotary motion from proximal end 234 of curved reamer 226 to reamer head 230 to effect reaming into femoral head 114 as illustrated in Figure 9. Flexible driveshaft 240 may include various flexible cuts, including the flexible cuts described in U.S. Patent No. 6,053,922. Guide tube 228 of curved femoral head reamer 226 includes curved guide channel 238 for guiding movement of outer tube 242 of reamer shaft 232 as reamer head 230 is advanced into femoral head 114 as illustrated in Figure 9. Curved femoral shaft reamer 242 has an identical structure to curved femoral head reamer 226 and, therefore, is not illustrated in detail for the sake of brevity. In an exemplary embodiment of the present invention, the head of curved femoral shaft reamer 242 is larger than the head of curved femoral head reamer 226. Similarly, the head of curved femoral head reamer 226 may be larger than the head of curved femoral shaft reamer 242. Moreover, the radius of curvature of the reamer shafts may differ between curved femoral head reamer 226 and curved femoral shaft reamer 242. In all cases, a tubular reamer shaft and flexible driveshaft is utilized.

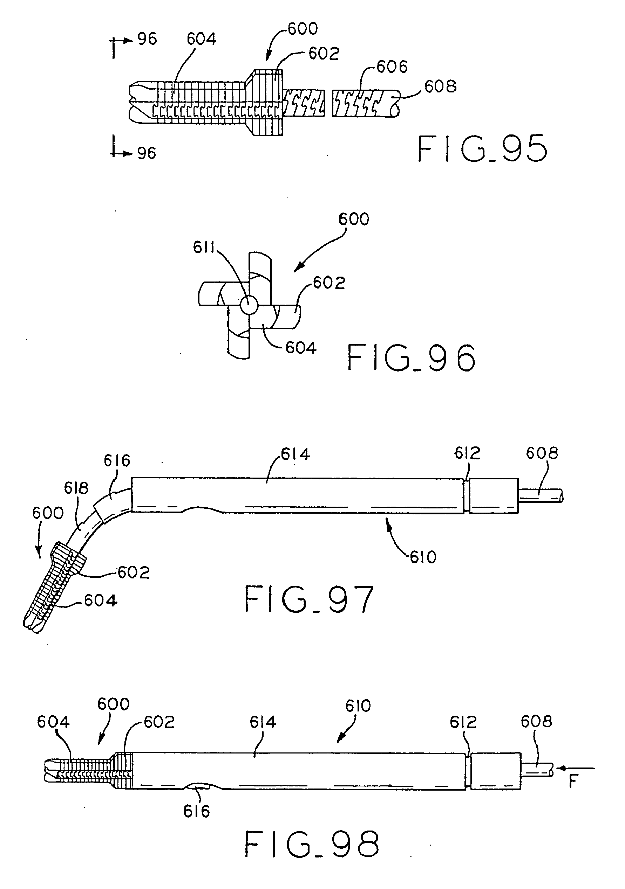

Telescoping reamer 610 illustrated in Figures 97-99 maybe utilized in lieu of curved femoral head reamer 226 and/or curved femoral shaft reamer 242. While illustrated in Figures 97-99 with a flex up reamer head (described below), telescoping reamer 610 may be utilized with other reaming heads including, e.g., a reaming head adapted for extending the implant cavity distally into the intramedullary canal of the femoral shaft. Referring to Figures 97-99, telescoping reamer 610 includes body 614 having detent groove 612 formed in an exterior thereof. Detent groove 612 is useful for receiving the ball detent of the ball detent retaining mechanism described below, although body 614 may include any of the mechanisms disclosed herein for positioning and/or locking an instrument into any of the guide tube/retractors of the present invention.

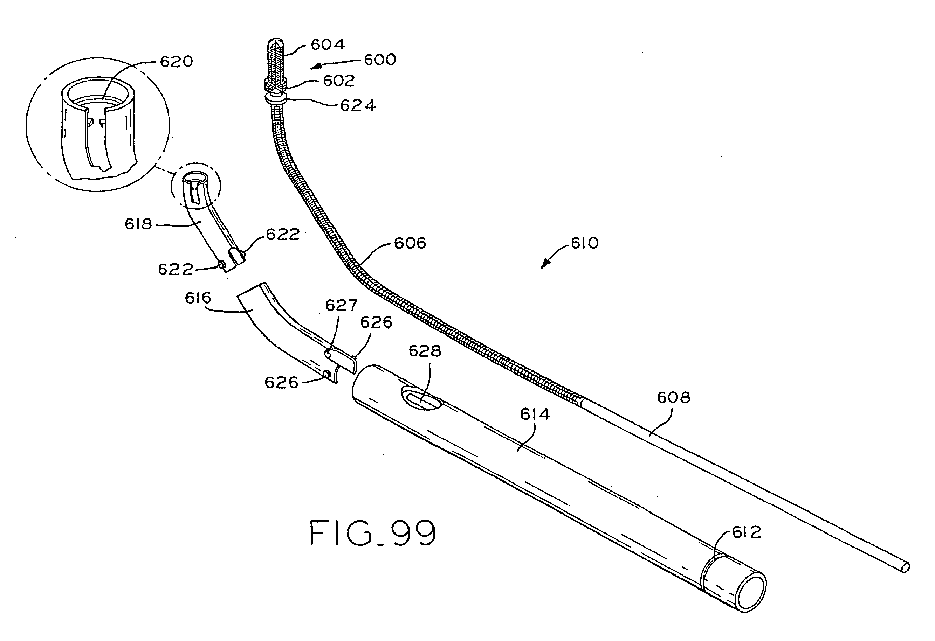

Referring to Figure 99, in construction, outer extension sleeve 616 is positioned within body 614 of telescoping reamer 610, with exterior bosses 626 of outer extension sleeve 616 positioned within internal channels 628 (only one of which is depicted in Figure 99) of body 614. Similarly, inner extension sleeve 618 is positioned within outer extension sleeve 616, with exterior bosses 622 of inner extension sleeve 618 positioned within internal channels 627 (only one of which is depicted in Figure 99) of outer extension sleeve 616. Internal channels 627 and 628 of outer extension sleeve 616, and body 614, respectively, guide the direction and extent of relative movement between inner extension sleeve 618 and outer extension sleeve 616, and outer extension sleeve 616 and body 614, respectively. Both channels 627 and 628 have proximal and distal ends. When bosses 622, and 626 are positioned adjacent the proximal ends of channels 627 and 628, respectively, telescoping reamer 610 maintains the retracted position illustrated in Figure 98. Similarly, when bosses 622 and 626 abut the distal ends of channels 627and 628, respectively, telescoping reamer 610 maintains the extended position illustrated in Figure 97.

As illustrated in Figures 97-99, body 614 of telescoping reamer 610 includes a cutout accommodating the proximal end of outer extension sleeve 616 when telescoping reamer 610 maintains the retracted position illustrated in Figure 98. In construction, flexible reamer shaft 606 is positioned within inner extension sleeve 618 and, consequently, within outer extension sleeve 616 and body 614. The reamer shaft runs the length of body 614, with straight reamer shaft 608 extending from a distal end thereof. As illustrated in Figure 99, flange 624 is positioned about flexible reamer shaft 606 and spaced from the proximal portion of large diameter portion 602 of flex up reamer 600 (further described hereinbelow). In construction, interior flange 620 of inner extension sleeve 618 is positioned intermediate large diameter portion 602 of flex up reamer 600 and flange 624 extending from flexible reamer shaft 606.

To extend telescoping 610 reamer from the non-extended position illustrated in Figure 98 to the extended position illustrated in Figure 97, force F (Figure 98) having a vector component parallel to the longitudinal axis of straight reamer shaft 608 is applied to straight reamer shaft 608, placing flange 624 in abutting relationship with interior flange 620 of inner extension sleeve 618. As additional force is applied to straight reamer shaft 608, the abutting relationship of flange 624 and interior flange 620 causes extension of inner extension sleeve 618 outwardly from outer extension sleeve 616 and, consequently, body 614. Inner extension sleeve 618 extends from outer extension sleeve 616 until bosses 622 abut the distal ends of internal channels 627 of outer extension sleeve 616. In this position, additional force applied to straight reamer shaft 608 causes extension of outer extension sleeve 616 out of body 614. Outer extension sleeve 616 extends until exterior bosses 626 abut the distal ends of internal channels 628 of body 614. In this position, telescoping reamer 610 is fully extended as illustrated in Figure 97. Inner extension sleeve 618 and outer extension sleeve 616 may be formed with various curvatures accommodating reaming from greater trochanter 110 into femoral head 114, as well as reaming from greater trochanter 110 into the intramedullary canal of femur 108.

To retract telescoping reamer 610 from the extended position illustrated in Figure 97 to the non-extended position illustrated in Figure 98, straight reamer shaft 608 is pulled in a generally opposite direction to force F illustrated in Figure 98. When straight reamer shaft 608 is pulled in this manner, the reamer head pulls inner extension sleeve 618 into outer extension sleeve 616 until bosses 622 abut the proximal ends of internal channels 627 of outer extension sleeve 616. In this position, additional pulling of straight reamer shaft 608 pulls outer extension sleeve 616 into body 614 until telescoping reamer 610 achieves the non-extended position illustrated in Figure 98.

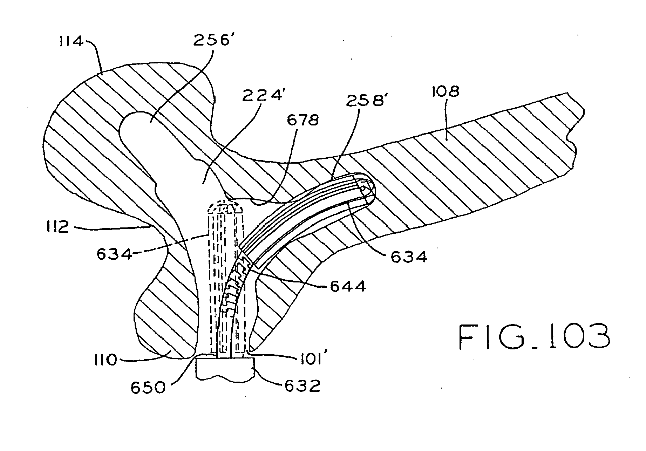

In use, telescoping reamer 610 is inserted through incision 106 and secured within a guide tube/retractor of the present invention. Telescoping reamer 610 may be utilized to form access 101 in femur 108 in lieu of straight reamer 186 illustrated in Figure 6. Alternatively, straight reamer 186 may be utilized to form access 101 in femur 108 prior to insertion of telescoping reamer 610 through incision 106. In any event, after straight reaming is complete and access 101 is formed in femur 108 as illustrated in Figure 6, telescoping reamer 610 is oriented whereby extension of telescoping reamer 610 from the non-extended position illustrated in Figure 98 to the extended position illustrated in Figure 97 extends implant cavity 224' into femoral head 114, forming femoral head arm 256' of implant cavity 224' as illustrated in Figure 103. In certain embodiments, telescoping reamer may be reoriented to extend from greater trochanter 110 into the intermedullary canal of femur 108 to form femoral shaft arm 258' of implant cavity 224'. In such an embodiment, telescoping reamer 610 will not include a reamer head having a pair of reaming diameters as illustrated in Figures 97-99.

After formation of femoral cavity 224, any remaining guide tube/retractor as well as guide plate 126 is removed and implant 260 is positioned through access 101 to be implanted in femoral cavity 224. During implantation of implant 260, retractors are utilized to provide access from incision 106 to access 101 formed in femur 108. As illustrated in Figure 12, bag 270 (Figure 41) is manipulated into a relatively small package positioned adjacent lag screw tube 266 before inserting implant 260 through access 101. In one exemplary embodiment, bag 270 is accordion folded. As further illustrated in Figure 12, fill tube 262 and reinforcement/expansion bar 268 of femoral implant 260 are positioned adjacent lag screw tube 266 for positioning implant 260 through access 101 and into femoral cavity 224. When femoral implant 260 is fully inserted through access 101, lag screw thread 282 abuts the entry to femoral head arm 256 of implant cavity 224 as illustrated, e.g., in Figure 13. In this position, fill tube 262 and reinforcement/expansion bar 268 can be manipulated into the operable position illustrated in Figure 14. In this position, bag 270 extends into femoral shaft arm 258 of implant cavity 224.

After implant 260 is positioned as illustrated in Figure 13, a flexible drive device is utilized to advance lag screw 264 into femoral head 114 until reaching the terminal position illustrated in Figure 14. With lag screw 264 firmly implanted in femoral head 114, pump P is utilized to convey a bag fill material for filling bag 270 from source of bag fill 284 through fill tube 262. In one exemplary embodiment, source of bag fill 284 comprises a source of bone cement. Fill tube 264 is formed to provide for retrograde filling of bag 270. As bag 270 is filled with, e.g., bone cement, it expands to fill femoral cavity 224, including, femoral shaft arm 258 thereof. Once bag 270 is filled, the bone cement injected therein cures and provides intramedullary fixation of femoral implant 260. As indicated above, in a further embodiment of the present invention, the bag structure of the implant of the present comprises a nested bag structure in which an inner bag is filled with a high strength material relative to an outer bag in which the inner bag is placed. The outer bag of this form of the present invention is formed from and filled with a more bioresorbable material relative to the material of construction and fill material of the inner bag.

Implant 260 is illustrated in detail in Figure 41. As illustrated in Figure 41, bag 270 is secured to lag screw tube 266 to prevent material inserted into bag 270 from escaping between the contact points formed between bag 270 and lag screw tube 266. As further illustrated in Figure 41, reinforcement/expansion bar 268 is positioned to facilitate deployment of implant 260 into femoral shaft arm 258 of femoral cavity 224 as described hereinabove. Reinforcement/expansion bar 268 will not be utilized in every embodiment of the present invention. As illustrated in Figure 43, reinforcement/expansion bar 268 also functions to laterally spread bag 270 to facilitate placement of bone cement therein. As illustrated in Figure 41, fill tube 262 is positioned within bag 270, with bag 270 securely affixed to a proximal end thereof.

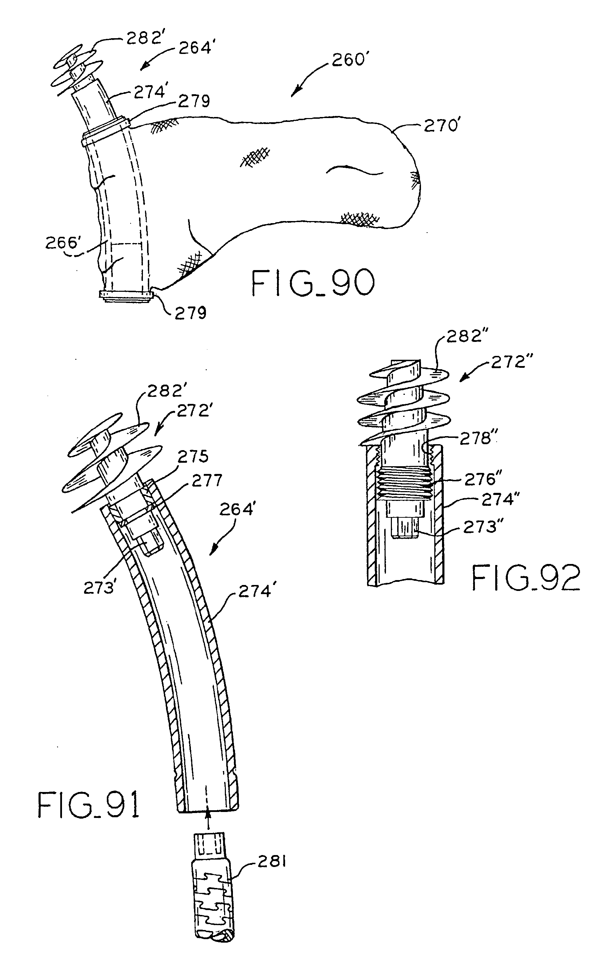

Figure 90 illustrates alternative embodiment femoral implant 260'. Femoral implant 260' is generally identical to femoral implant 260 illustrated in Figure 41 except for the provision of external fasteners 279 utilized to securely affix bag 270' to lag screw tube 266. Although not illustrated in Figure 90, it is contemplated that femoral implant 260' will include a fill tube 262' for filling bag 270 with bone cement. Bag 270 of femoral implant 260 can be, e.g., formed of various films and fabrics. In one exemplary embodiment, bag 270 is formed from an acrylic material, e.g., a woven acrylic material. Because bone cement is an acrylic, if implant bag 270 is formed of an acrylic material, implant bag 270 and the bone cement will achieve an intimate chemical bond. Implant bag 270 of femoral implant 260 of the present invention generally comprises a containment device and can be constructed of various materials including films such as, e.g., fiber or fabric reinforced films, or fabrics created by processes such as weaving, knitting, braiding, electrospinning, or hydrospinning. Alternative materials contemplated for implant bag 270 include various polymers including, e.g., polymethylmethacrylate, polycarbonate, UHMWPE, LDPE, HDPE, polyamides, polypropylene, polyester, polyaryletherketone, polysulfone, or polyurethane. Further alternative materials contemplated for implant bag 270 include fabrics constructed of fibers formed of glass, ceramics, surgical grade stainless steel (e.g., 316L), titanium, or titanium alloys. Moreover, implant bag materials may be coated with, e.g., calcium phosphate, or a bioactive glass coating. Furthermore, implant bag 270 and the associated filler may be utilized as a delivery mechanism for, e.g., drugs, or growth factors.

Alternative embodiments of the lag screw of the present invention are illustrated in Figures 42,91, and 92. As illustrated in Figure 42, lag screw 264 generally comprises curved lag screw shaft 274 rotatably connected to lag screw head 272. In the embodiment illustrated in Figure 42, lag screw shaft 274 includes distal male threads 276 cooperating with proximal female threads 278 formed in lag screw head 272. Mating threads 276, 278 are left handed threads. Lag screw head 272 includes chamber 280 to accommodate distal threaded end 276 of lag screw shaft 274 when lag screw head 272 is operably positioned on lag screw shaft 274. Lag screw head 272 includes distal lag screw threads 282 for implanting lag screw 264 into femur 108 as described hereinabove. Cooperating threads 276, 278 are left handed threads, while lag screw threads 282 are right handed threads. In this way, lag screw head 272 may be threadedly engaged on lag screw shaft 274 and, rotation of lag screw head 272 in a clockwise fashion to effect implantation of lag screw threads 282 into femur 108 will not cause lag screw head 272 to become separated from lag screw shaft 274.

Figure 91 illustrates alternative embodiment lag screw 264' in which lag screw head 272 includes flange 277 and lag screw shaft 274 includes bearing protrusion 275. In this embodiment, bearing protrusion 275 is positioned intermediate the most proximal portion of lag screw head 272' and flange 277. In this arrangement, flange 277 cooperates with the most proximal portion of lag screw head 272 and bearing protrusion 275 to prohibit axial displacement of lag screw head 272'. Lag screw head 272' includes male hex 273' operable for connection to flexible drive 281 as illustrated in Figure 91. In use, flexible drive 281 will be inserted within tubular lag screw shaft 274 and engaged with male hex 273' to rotate lag screw head 272 to effect implantation thereof. In the embodiment illustrated in Figure 42, lag screw shaft 274 is similarly canulated to allow a flexible drive to enter lag screw shaft 274 and engage a cooperating protrusion (not shown) formed on lag screw head 272. Figure 92 illustrates an alternative embodiment of lag screw head 272" wherein male threads 276" are formed on lag screw head 272", and female threads 278' are formed in lag screw shaft 274.

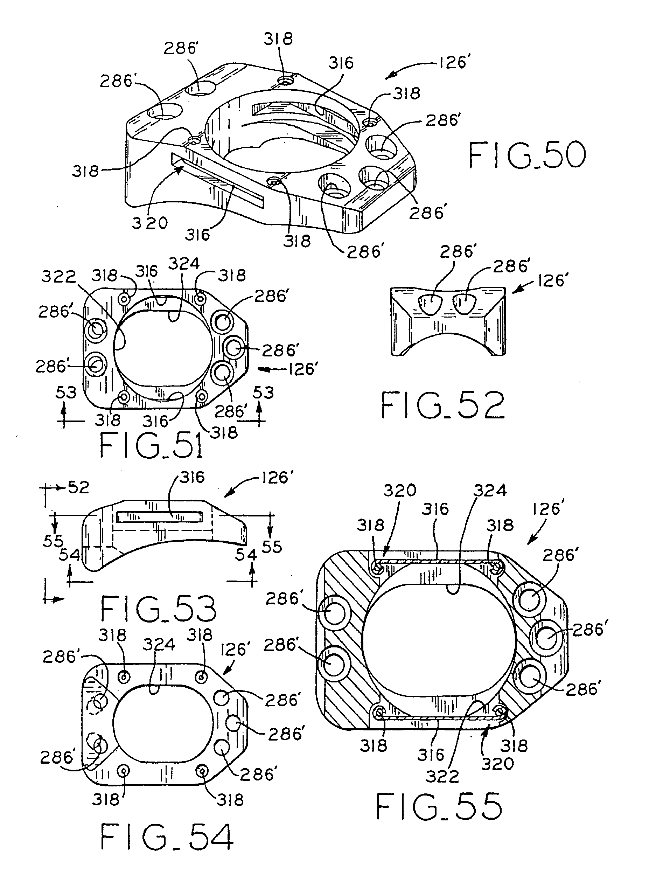

Alternative embodiments of guide plate 126 are illustrated in Figures 50-55, and 65-68. Referring now to Figures 50-55, guide plate 126' includes screw apertures 286' for use in securing guide plate 126 to femur 108 as described hereinabove with respect to guide plate 126. Guide plate 126' further includes spring pins 318 traversing axially oriented apertures in guide plate 126'. As illustrated in Figure 55, spring pins 318 engage alternate ends of springs 316 to hold springs 316 in position within guide plate 126'. As illustrated in Figure 51, guide plate 126' includes circular opening 322 as well as elliptical opening 324, with springs 316 extending into circular opening 322. In one exemplary embodiment, springs 316 are formed from titanium.

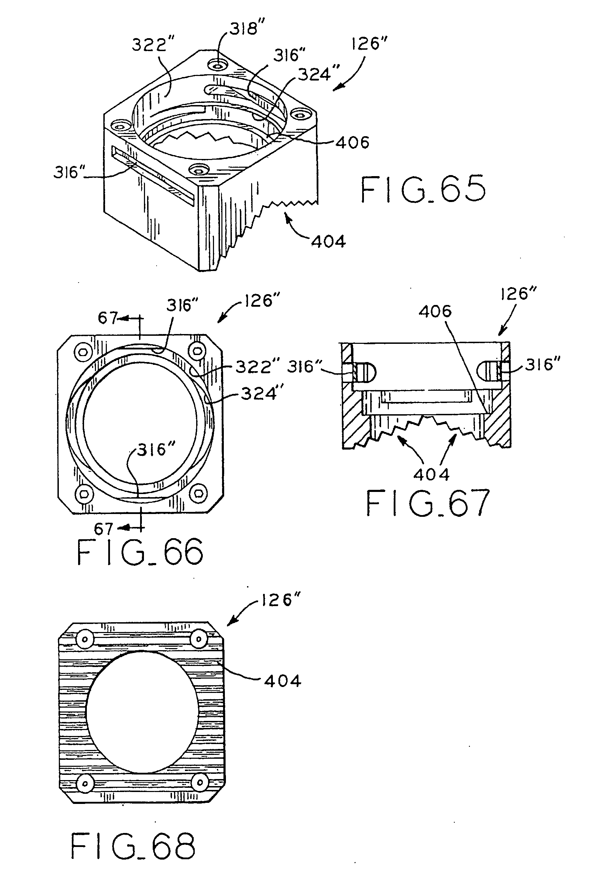

Referring now to Figures 65-68, guide plate 126" includes axially oriented apertures accommodating spring pins 318" in much the same way as guide plate 126' illustrated in Figures 50-55. Spring pins 318" are utilized to hold springs 316" in position within guide plate 126" as illustrated with respect to guide plate 126' in Figure 55. Guide plate 126" includes circular opening 322" as well as elliptical opening 324" similar to the corresponding openings found in guide plate 126'. The distal end of guide plate 126" includes gripping teeth 404 formed thereon. Additionally, guide plate 126" includes fixation screw shoulder 406 as illustrated, e.g., in Figure 67. Fixation screw shoulder 406 will be further described hereinbelow.

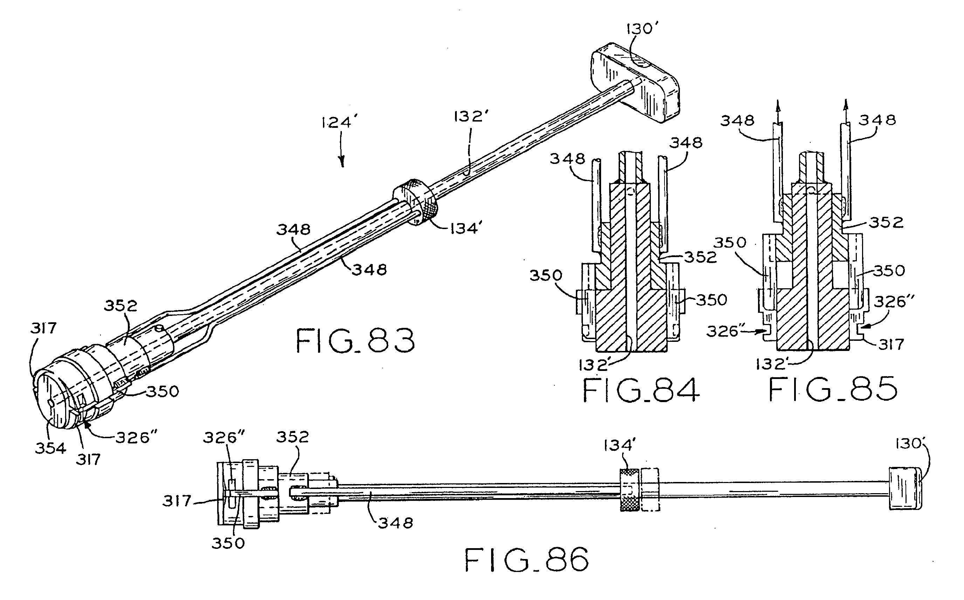

In use, guide plate 126' is inserted through incision 106 for affixation to femur 108 in the same manner as guide plate 126 described hereinabove. Insertion member 124' illustrated in Figures 83-86 is utilized to position guide plate 126' through incision 106 for placement atop greater trochanter 110. In many respects, insertion instrument 124' is similar to insertion instrument 124 illustrated in Figures 19-22 and further described hereinabove. As illustrated in Figures 83-86, insertion instrument 124' includes elongate aperture 132' for accommodating stabilization nail 144 (Figure 2). Insertion member 124' includes release member 134' connected via connecting rods 348, and cylindrical connector 352 to release bars 350. Release bars 350 travel in axially oriented slots formed in the distal end of insertion member 124. The distal end of insertion member 124' includes elliptical protrusion 354 for placement within elliptical aperture 324 of guide plate 126'. Cooperation of elliptical protrusion 354 with elliptical aperture 324 insures proper rotational alignment of insertion member 124' and guide plate 126'. Upon achieving proper rotational alignment, insertion member 124' may be axially displaced into the central aperture of guide plate 126', with springs 316 engaging spring slots 326" formed in opposing sides of the distal end of insertion member 124'. In this way, springs 316 lock guide plate 126' to insertion member 124'. Bevel 317 facilitates positioning of springs 316 in spring slots 326". After guide plate 126' is secured to femur 108 as described hereinabove with respect to guide plate 126, release bars 350 are utilized to actuate springs 316 radially outwardly from their normally biased position to disengage spring slots 326" and allow for removal of insertion member 124' from guide plate 126'.

Release member 134' is utilized to effect axial displacement of release bars 350 from the position illustrated in Figure 85 in which spring slots 326" are available for engagement with springs 316 to the position illustrated in Figure 84 in which release bars 350 provide a radially outward force to springs 316 to allow for disengagement of insertion member 124' from locking engagement with guide plate 126' and allow for removal of insertion member 124' through incision 106. As illustrated in Figure 85, release bars 350 include a distal bevel to facilitate movement from the position illustrated in Figure 85 to the position illustrated in Figure 84 to effect release of springs 316 from spring slots 326". Similarly, insertion member 124' can be lockingly engaged with guide plate 126" illustrated in Figures 65-68 to effect implantation of guide plate 126" through incision 106 for placement atop greater trochanter 110.

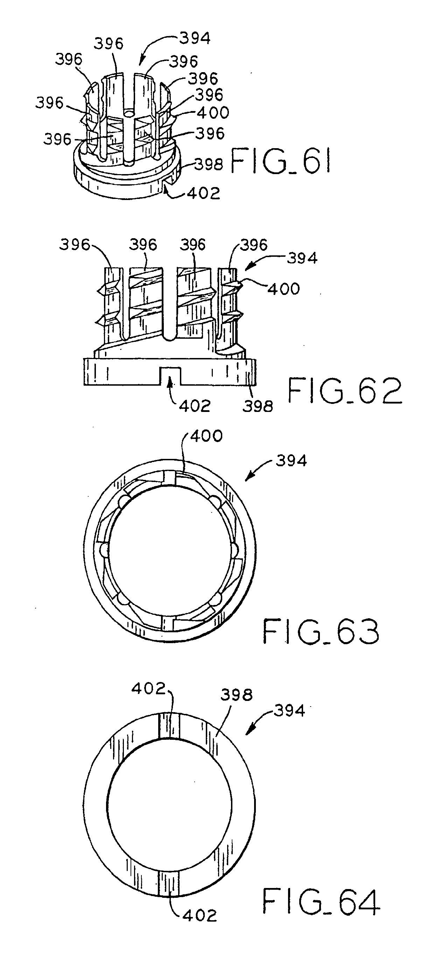

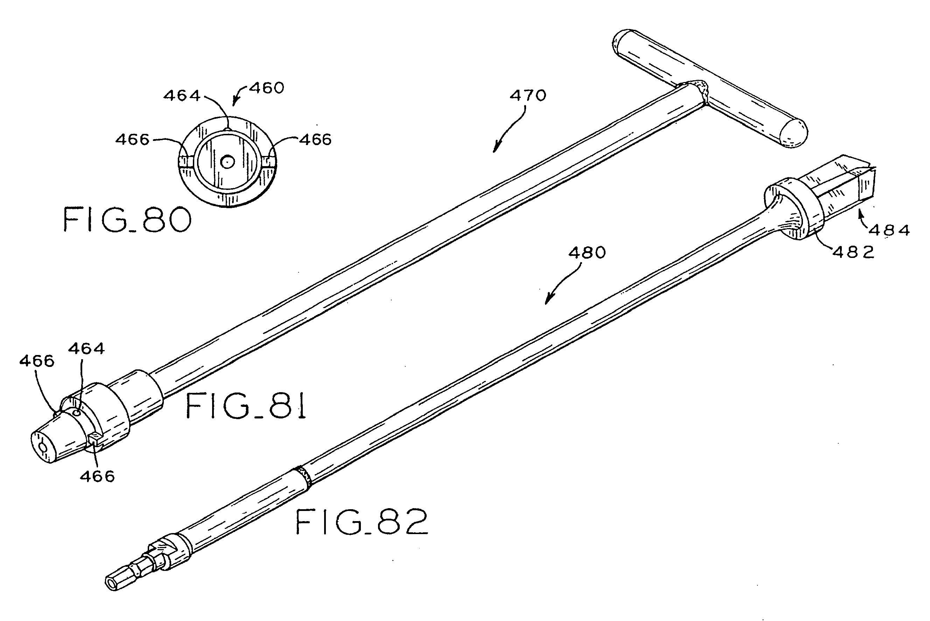

When utilizing guide plate 126" illustrated in Figures 65-68, plunge reamer 480 (Figure 82) must first be utilized to form a cavity in femur 108 extending through greater trochanter 110. Plunge reamer 480 includes reamer head 484 and flange 482. In this embodiment, plunge reamer 480 is inserted through incision 106 and reamer head 484 is placed atop greater trochanter 110. As with initial placement of guide plate 126 and 126', a fluoroscope may be utilized to facilitate proper positioning of reamer head 484 atop greater trochanter 110. Furthermore, a surgeon may rely on tactile feedback for proper positioning of plunge reamer 480. Plunge reamer 480 is actuated and plunge reaming is effected until flange 482 abuts greater trochanter 110. Plunge reamer 480 is thereafter removed through incision 106 to allow for placement of guide plate 126" atop greater trochanter 110. Fixation screw 394 illustrated in Figures 61-64 is thereafter utilized to secure guide plate 126" to greater trochanter 110. While insertion instrument 124' may be utilized to initially position guide plate 126" through incision 108, it must be removed prior to implantation of fixation screw 394.

As illustrated in Figures 61-64, fixation screw 394 includes fixation screw head 398 with fingers 396 axially depending therefrom. Screw threads 400 are formed on axially extending fingers 396. The proximal end of fixation screw 394 includes locking channel 402, the utility of which will be further described hereinbelow. Fixation screw head 398 forms a flange engagable with fixation screw shoulder 406 formed in guide plate 126" (Figure 67). Fixation screw 394 is inserted through the central aperture of guide plate 126" and is screwed into the bore formed through greater trochanter 110 to secure guide plate 126" atop greater trochanter 110. Threads 400 cut into the femoral bone stock to provide fixation of fixation screw 394.

Fixation screw placement instrument 470 as illustrated in Figures 80 and 81 is utilized to insert fixation screw 394 through incision 106 and to secure fixation screw 394 within guide plate 126" as described hereinabove. Referring now to Figures 80 and 81, fixation screw placement instrument 470 includes a proximal handle as well as a distal end having blades 466 and ball detent 464 formed therein. In use, blades 466 engage locking channels 402 in fixation screw 394, with ball detent 464 engaging a detent (not shown) formed in the inner diameter of locking screw 394. The proximal handle of fixation screw placement instrument 470 may then be utilized to rotate fixation screw 394 and secure the same within femur 108.

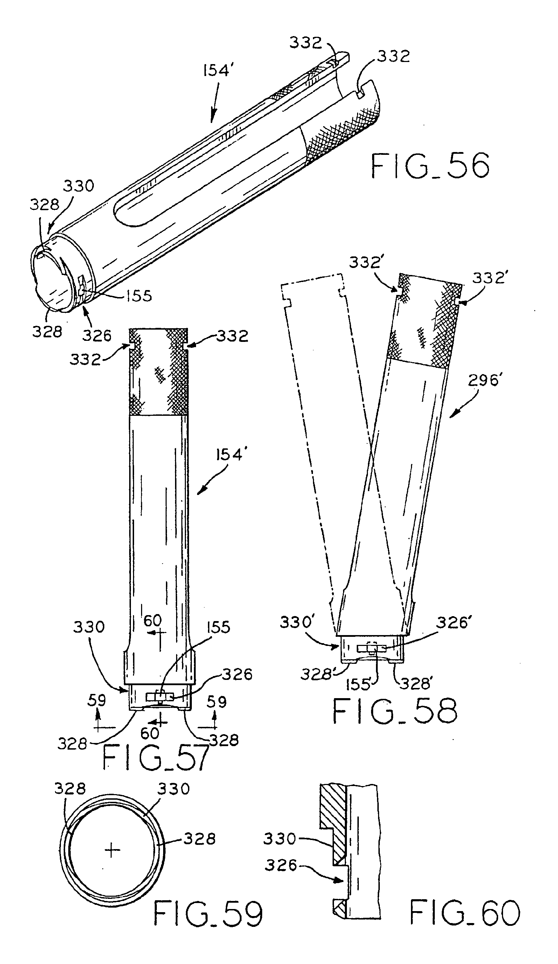

When utilizing either guide plate 126' (Figures 50-55) or guide plate 126" (Figures 65-68), alternative embodiment guide tube/retractor 154' is utilized in lieu of guide tube/retractor 154 described hereinabove with reference to guide plate 126. Guide tube/retractor 154' is illustrated in Figures 56, 57, 59, and 60. As illustrated, guide tube/retractor 154' includes a distal end having rounded portion 330 with spring slots 326 formed on opposing sides thereof. Furthermore, distal end of guide tube/retractor 154' includes engagement protrusions 328 having a radius of curvature matching the rounded ends of elliptical openings 324 and 324" in guide plates 126' and 126", respectively. Opposing spring slots 326 formed in the distal end of guide tube/retractor 154' are utilized to selectively affix guide tube/retractor 154' to either guide plate 126' or 126" in the same fashion as described above with respect to insertion member 124'. As illustrated in Figure 58, angled guide tube/retractor 296' is provided for use with guide plates 126' or 126". Angled guide tube/retractor 296' provides the same functionality as angled guide tube/retractor 296 described hereinabove with respect to guide plate 126 and includes a distal end identical to the distal end of straight guide tube/retractor 154 illustrated in Figures 56, 57, 59, and 60. Straight guide tube/retractor 154' and angled guide tube/retractor 296' have a greater axial length than straight guide tube/retractor 154 and angled guide tube/retractor 296 described in the primary embodiment of the present invention. The inventors of the present invention contemplate various guide tube/retractors having differing lengths to accommodate physiological differences in a variety of patients as well as different attaching mechanisms in accordance with the various embodiment of the present invention. As illustrated in Figures 56-60, guide tube/ retractors 154' and 296' include latch channels 332 and 332', respectively. The utility of latch channels 332 and 332' will be further described hereinbelow.

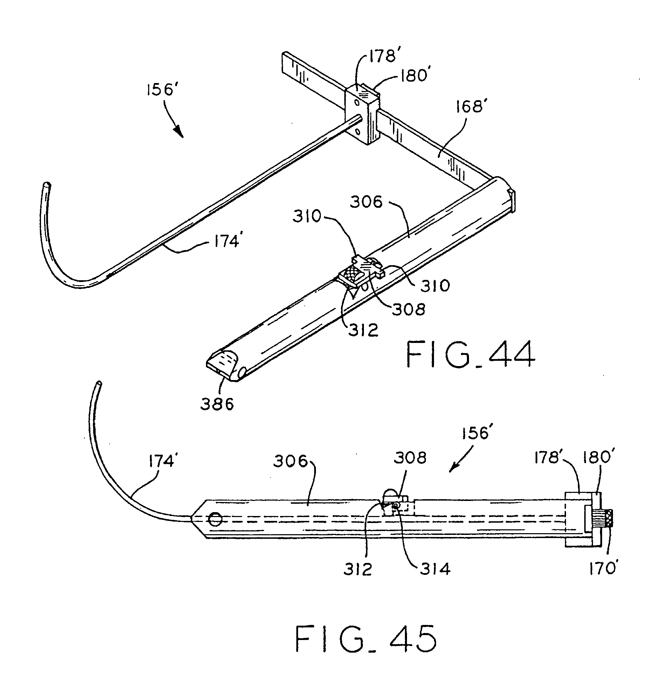

Referring now to Figures 44 and 45, alignment device 156' is utilized in conjunction with guide tube/retractors 154', 296'to select the appropriate guide tube/retractor as described hereinabove with respect to alignment device 156. Alignment device 156' includes alignment guide tube 306 for positioning within guide tube/retractor 156', or angled guide tube/retractor 296' and providing a stable base for alignment device 156' as described above with respect to insertion portion 160 of alignment device 156 (Figures 29 and 30). Alignment guide tube 306 includes latch 308 pivotally connected thereto via pivot pin 314. Additionally, alignment guide tube 306 includes distal flat 386 which, in this exemplary embodiment will bottom out on the shoulder formed between the elliptical aperture and a round aperture in guide plates 126' and 126". Latch 308 includes oppositely depending locking tabs 310 extending from opposing sides thereof. Latch 308 is biased into the position illustrated in Figure 45 by spring 312. As alignment guide tube 306 is inserted into guide tube/retractor 156' or 296', locking tabs 310 contact the proximal end of guide tube/retractor 154' or 296'. After achieving this position, the distal end of latch 308 is depressed radially inwardly to move locking tabs 310 away from alignment guide tube 306 and allow for further insertion of alignment guide tube 306 into guide tube/retractor 154' or angled guide tube/retractor 296'. As indicated above, distal flat 386 bottoms out on the shoulder formed between the elliptical and the round apertures in guide plates 126' and 126" when alignment guide tube 306 is fully inserted into guide tube/retractor 154' or 296'. In this position, locking tabs 310 align with latch channels 332 (Figures 56-58) and latch 308 can return to its normally biased position as illustrated in Figure 45. In this position, locking tabs 310 engage latch channels 332 to prevent axial displacement of alignment guide tube 306 relative to guide tube/retractor 154' or 296'. Furthermore, when engaged in latch channels 332, locking tabs 310 resist rotational movement of alignment guide tube 306. In all other respects, alignment device 156' is identical to alignment device 156 described above and is utilized in a similar fashion to choose between straight guide tube/retractor 154' and angled guide tube/retractor 296'.

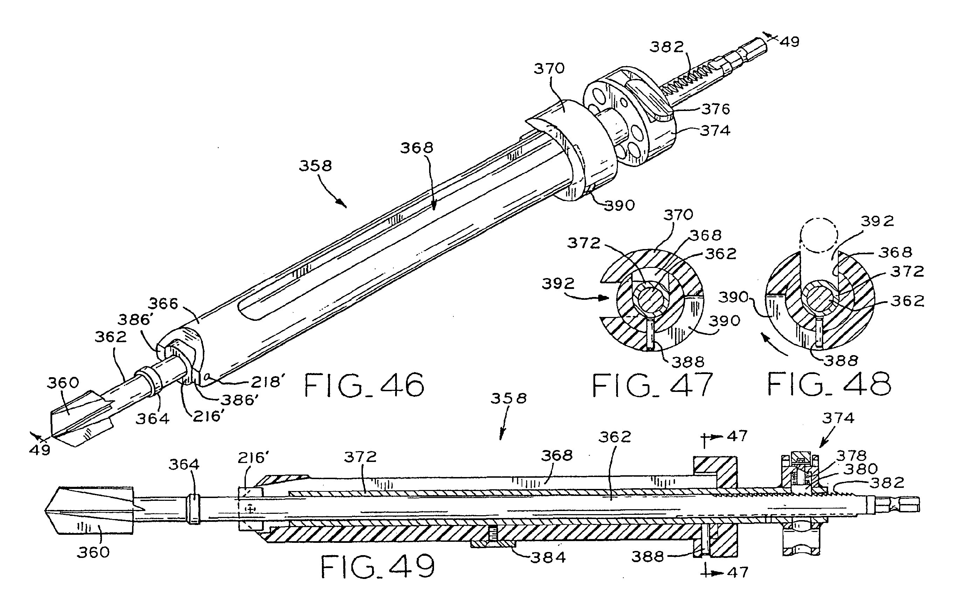

Reaming of femoral cavity 224 is effected with reamers having guide tubes and latches similar to guide tube 306 and latch 308 described above with respect to alignment device 156'. In one alternative embodiment, combination reamer 358 illustrated in Figures 46-49 is utilized to effect both plunge, i.e., straight reaming into the femur as well as swivel reaming. In this embodiment, combination reamer 358 is inserted into guide tube/retractor 154' or 296', with orientation plate 384 cooperating with one of the longitudinal channels formed in guide tube/retractor 154' or 296' (see, e.g., Figures 56-60) to properly align combination reamer 358 within the guide tube/retractor. As illustrated in Figures 46-49, combination reamer 358 includes reamer head 360 connected to the distal end of reamer shaft 362. Reamer shaft 362 includes flange 364 positioned toward the distal end thereof and ratchet teeth 382 formed toward the proximal end thereof. As illustrated in Figure 49, reamer shaft 362 is positioned within reamer shaft tube 372 having reamer depth lock 374 formed on a proximal end thereof. Reamer depth lock 374 includes ratchet release 376 connected via connecting rod 378 to ratchet head 380 as illustrated in Figure 49. As illustrated in Figure 49, a spring is utilized to bias ratchet head 380 into engagement with ratchet teeth 382 on reamer shaft 362. Ratchet release 376 is pivotally connected to reamer depth lock 374 such that actuation of ratchet release 376 causes outward radial movement of ratchet head 380 with respect to reamer shaft 362, thus disengaging the ratchet teeth formed in ratchet head 380 from ratchet teeth 382 and allowing for relative axial movement of reamer shaft tube 372 and reamer shaft 362. In the configuration illustrated in Figure 49, combination reamer 358 can be utilized to effect plunge reaming, with the terminal reaming depth being reached when the distal end of reamer shaft tube 362 contacts pivot 216. The overall depth of plunge reaming may thus be adjusted by varying the axial displacement of reamer depth lock 374 along reamer shaft 362.

As illustrated in Figure 46, combination reamer 358 includes combination reamer guide tube 366 having channel 368 formed therein. Swivel/plunge reaming selector 370 is operably connected to a proximal end of combination reamer guide tube 366. As illustrated in Figure 49, rotation guide pin 388 is fixably secured to combination reamer guide tube 366 and positioned within rotation guide channel 390 of swivel/plunge reaming selector 370. Swivel/plunge reaming selector 370 may be rotated about guide tube 366 of combination reamer 358 between the extremes illustrated in Figures 47 and 48, i.e. with rotation guide pin 388 abutting opposite ends of rotation guide channel 390. When swivel/plunge reaming selector 370 is positioned as illustrated in Figure 47, swivel reaming with combination reamer 358 is not allowed because swivel/plunge reaming selector 370 covers channel 368. To allow for swivel reaming, swivel/plunge reaming selector 370 is rotated into the position illustrated in Figure 48. In the position illustrated in Figure 48, channel 392 in swivel/plunge reaming selector 370 aligns with channel 368 in guide tube 366 of combination reamer 358. In this position, swivel reaming can be effected as illustrated in Figure 48. Reamer shaft 362 is connected to guide tube 366 of combination reamer 358 via pivot 216' and pivot pins 218' to allow for the swivel reaming illustrated in Figure 48. Combination reamer 358 includes distal flat 386' for signaling complete insertion of combination reamer 358 into reamer/guide tube 154' or 296'. As described above with respect to alignment guide tube 306 of alignment device 156', distal flat 386' bottoms out on the shoulder formed between the elliptical and round apertures in guide plates 126' and 126" when combination reamer 358 is fully inserted into guide tube/retractor 154' or 296'.

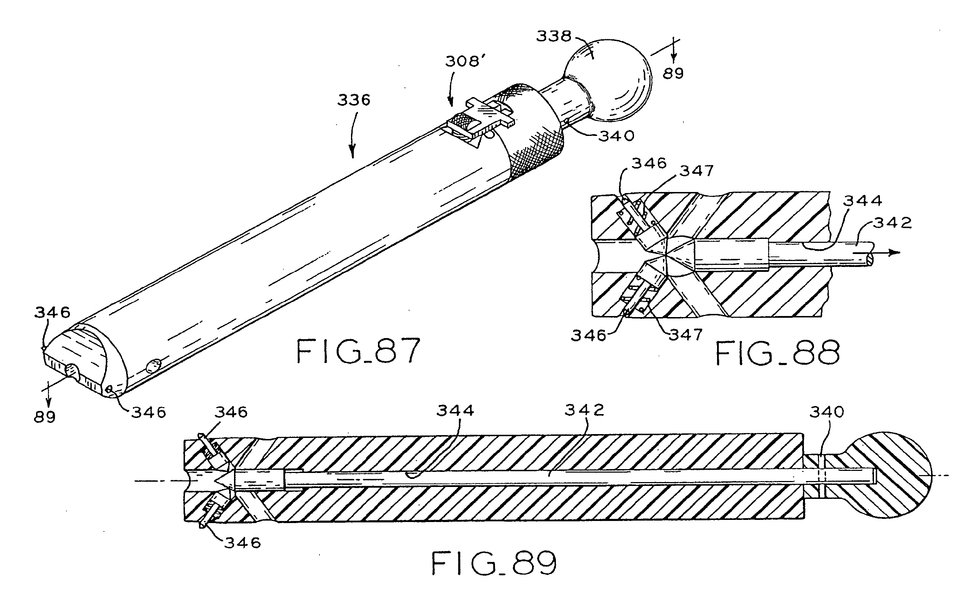

Upon completion of femoral reaming, guide tube/retractor 156' or 296' is removed from locked engagement with guide plate 126' or 126" with spring lock release instrument 336 illustrated in Figures 87-89. As illustrated in Figures 87-89, spring lock release instrument 336 includes a tubular body sized for insertion into guide tube/retractor 156' or 296' with a distal shoulder indicating complete insertion of spring lock release instrument 336 into guide tube/retractor 156' or 296' in the manner described above with respect to alignment guide tube 306 of alignment device 156', and combination reamer 358. Moreover, spring lock release instrument 336 includes latch 308' as described hereinabove with respect to guide tube 306 of alignment device 156'. After insertion of spring lock release instrument 336 into guide tube/retractor 156' or 296', handle 338 is utilized to axially displace actuation rod 342 traversing internal aperture 344 of spring lock release instrument 336 into the position illustrated in Figure 89. In this position, the distal ramped end of actuation rod 342 contacts the proximal ends of release pins 346 to overcome the biasing force of springs 347 (Figure 88) and cause release pins 346 to protrude from spring lock release instrument 336 as illustrated in Figure 89. In this position, release pins 346 traverse apertures 155, 155' and act against springs 316 to disengage springs 316 from spring slots 326 and allow for removal of guide tube/retractor 154' or 296'. In the embodiment illustrated, release pins 346 are spring biased. The inventors of the current invention contemplate that release pins 346 could be linked to actuation rod 346 via a mechanical linkage whereby pulling actuation rod 342 would pull pins 346 into the instrument and, conversely, pushing rod 342 would push the pins outwardly from the instrument. Moreover, while release pins 346 are illustrated as forming an acute angle with the longitudinal axis of spring lock release instrument 336, release pins 346 could be transversely positioned within spring lock release instrument 336.

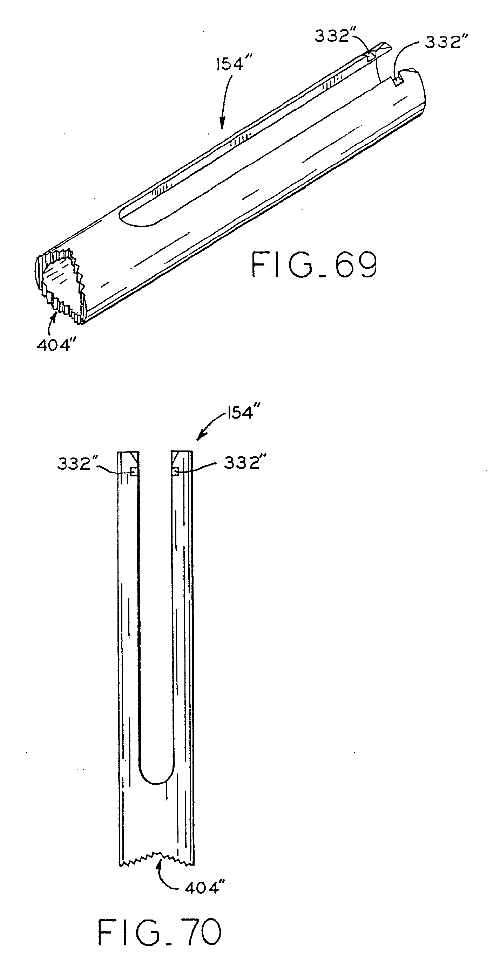

Guide tube/retractor 156" in accordance with a further alternative embodiment of the present invention is illustrated in Figures 69 and 70. In this embodiment, guide tube/retractor 154" is configured for affixation directly to greater trochanter 110, with guide plate 126 no longer being used. As illustrated in Figures 69 and 70, guide tube/retractor 154" includes gripping teeth 404" formed in a distal end thereof. In use, gripping teeth 404" are positioned atop greater trochanter 110 and fixation screw 394 is positioned within guide tube/retractor 154" and utilized to affix guide tube/retractor 154" to femur 108 as described above with reference to guide plate 126". While not illustrated in Figures 69 and 70, guide tube/retractor 154" includes a shoulder for engaging screw head 398 of fixation screw 394 to complete fixation of guide tube/retractor 154" to femur 108 in the same manner as described above with respect to guide plate 126".

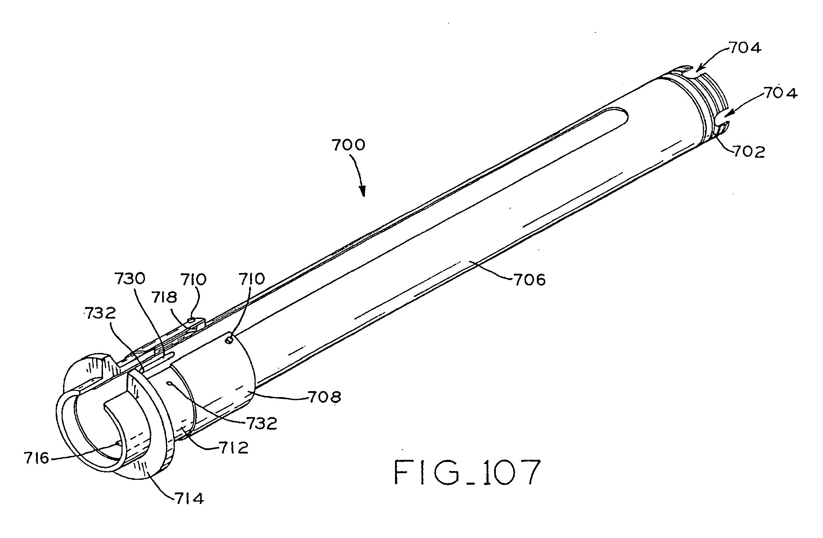

Figures 107-109 illustrate another alternative embodiment guide/retractor in accordance with the present invention. Specifically, Figures 107-109 illustrate unitube retractor 700. Unitube retractor 700 functions as the guide tube/retractors described above to maintain an access from incision 106 (Figure 1) made in the epidermis of patient 100 and developed to expose femur 108. Unitube retractor 700 is referred to as a "unitube" retractor because it is designed to be directly secured to femur 108, without use of a discrete guide plate or fixation screw. To effect fixation of unitube retractor 700 to femur 108, unitube retractor 700 includes self-tapping threads 702. Self-tapping threads 702 are formed on the distal end of unitube body 706, with cutouts 704 formed in and spaced about the periphery of the distal end of unitube body 706 to facilitate tapping of threads in femur 108 as unitube retractor 700 is threaded into engagement with femur 108 through access 101 described above. In an alternative embodiment, unitube retractor 700 will not include self-tapping threads, but rather will include threads that do not self-tap. In this embodiment, a discrete tap will be used to thread into access 101 in femur 108 prior to securement of unitube retractor 700 therein.

As illustrated in Figures 107-109, unitube body 706 includes a longitudinal slot to cooperate with guide tabs protruding from instruments to be inserted through unitube body 706 to properly align the instruments prior to use. The longitudinal slot formed in unitube body 706 will also accommodate the swivel reaming of certain embodiments of the present invention. In use, unitube retractor 700 will be inserted through incision 106 until the distal end abuts greater trochanter 110. In this position, a surgeon will utilize tactile feedback to position the distal end of unitube retractor 700 in access 101 formed in greater trochanter 110. In one exemplary embodiment, a fluoroscope will be utilized to facilitate positioning of the distal end of unitube retractor 700 in access 101 formed in greater trochanter 110. In this position, unitube retractor 700 will be threaded into access 101 in femur 108, with self-tapping threads 702 threading access 101 to secure unitube retractor 700 therein. Threading of unitube retractor 700 is complete when unitube retractor 700 is secured in access 101 and the longitudinal slot of unitube body 706 is aligned with an appropriate physiological landmark to guide alignment of instruments inserted therein. For example, a central axis of the longitudinal slot of unitube body 706 may be positioned substantially perpendicular to the plane of the greater trochanter and generally aligned with the axis of the femoral shaft.

As illustrated in Figures 107-109, unitube retractor 700 includes a ball detent retaining mechanism for retaining instruments inserted therein in a fixed longitudinal position relative to unitube body 706. The ball detent retaining mechanism cooperates with the longitudinal alignment slot of unitube body 706 to fix instruments positioned in unitube retractor 700 and prevent relative rotational and axial displacement of an instrument positioned in unitube retractor 700. Referring to Figures 107-109, ball detent 716 is received by counterbored ball detent aperture 720. Counterbored ball detent aperture 720 is formed from the exterior of unitube body 706 to the hollow interior thereof such that the largest diameter portion of counterbored ball detent aperture 720 is formed in the exterior wall of unitube body 706. Counterbored ball detent aperture 720 is sized whereby the smallest diameter portion thereof, i.e., the portion formed in the hollow interior of unitube body 706 is smaller than the equator of ball detent 716. With this structure, ball detent 716 cannot traverse counterbored ball detent aperture.

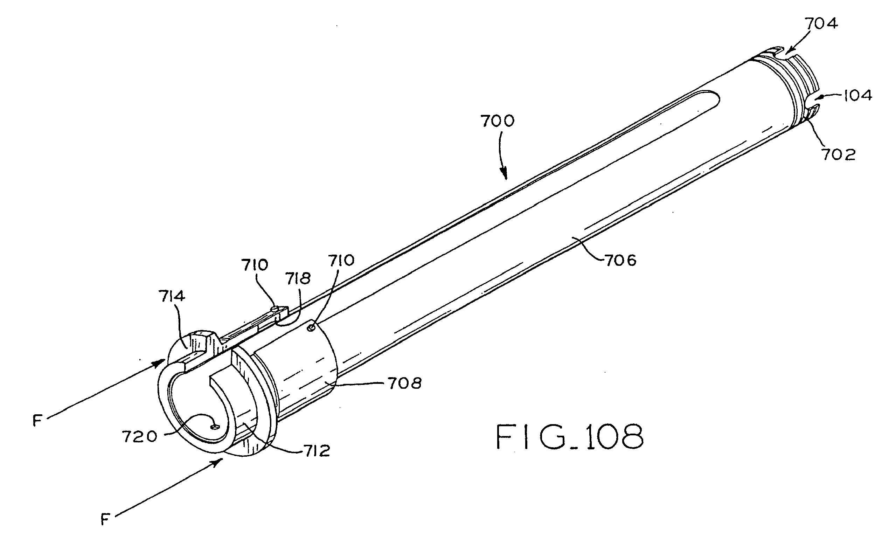

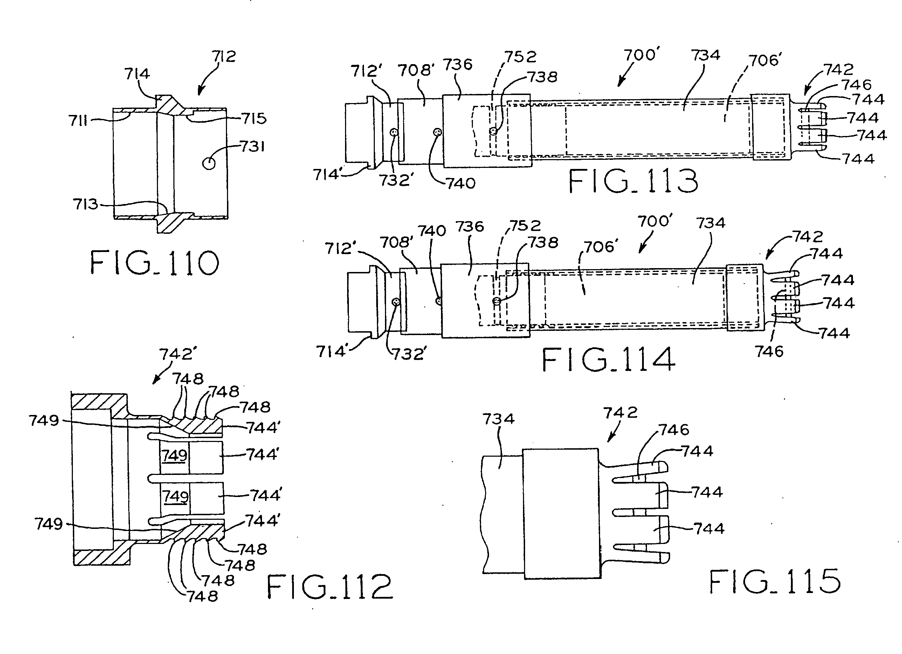

Ball detent 716 is interposed between plunger 712 and unitube body 706. As illustrated in Figure 110, plunger 712 includes internal ball detent ramp 713 connecting base flat 711 and peak flat 715. Figure 107 illustrates the ball detent retaining mechanism of unitube retractor 700 positioned to retain an instrument within unitube retractor 700, with ball detent 716 protruding into the hollow interior of unitube body 706. In this position, ball detent 716 contacts peek flat 715 (Figure 110) of plunger 712, which forces ball detent 716 to protrude into the hollow interior of unitube body 706. Figure 108 illustrates the ball detent retaining mechanism of unitube retractor 700 actuated to allow for release of an instrument positioned within unitube retractor 700, with ball detent 716 not protruding into the hollow interior of unitube body 706. In this position, ball detent 716 contacts base flat 711 (Figure 110) of plunger 712, which allows ball detent 716 to retract from the hollow interior of unitube body 706. As illustrated in Figure 108, force F is applied to flange 714 of plunger 712 to reposition plunger 712 from its normally biased position illustrated in Figure 107 to the position illustrated in Figure 108.

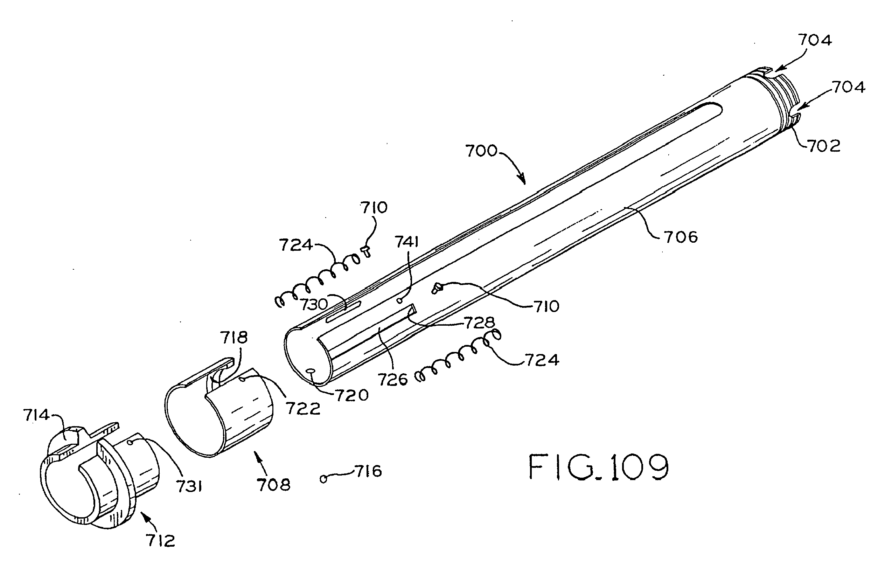

To bias plunger 712 into the position illustrated in Figure 107, springs 724 (Figure 109) are positioned intermediate plunger 712 and collar 708. Collar 708 includes internal collar flange 718 as illustrated in Figure 107-109. In construction, collar 708 is secured to unitube body 706 with set screws 710 positioned through set screw apertures 722 (only one of which is illustrated in Figure 109) in collar 708 and secured in set screw apertures 741 in unitube body 706. Springs 724 are positioned in spring slots 726 (only one of which is illustrated in Figure 109) on opposing sides of unitube body 706, with the distal ends of springs 724 abutting internal collar flange 718 and distal end 728 of spring slots 726. Spring slots 726 maintain the position of springs 724 substantially parallel to the longitudinal axis of unitube body 706. In one exemplary embodiment, internal collar flange 718 of collar 708 includes circular cutouts aligned with spring slots 726 to further facilitate alignment of springs substantially parallel to the longitudinal axis of unitube body 706. Plunger 712 is positioned over the proximal end of unitube body 706 such that springs 724 are interposed between internal collar flange 718 of collar 708 and the distal end of plunger 712. Plunger 712 includes at least one set screw aperture 731 and unitube body 706 includes at least one corresponding set screw slot 730. To complete assembly of unitube retractor 700, set screws 732 are threaded into set screw apertures 731 in plunger 712 and extend into set screw slots 730 in unitube body 706. Set screws 732 cooperate with set screw slots 730 to limit displacement of plunger 712 to longitudinal movement only. In the normally biased position illustrated in Figure 107, set screws 732 abut the proximal end of set screw slots 730. In use, ball detent 716 engages a detent formed in an instrument inserted into unitube retractor 700 to retain the instrument in a fixed position relative to unitube retractor 700.

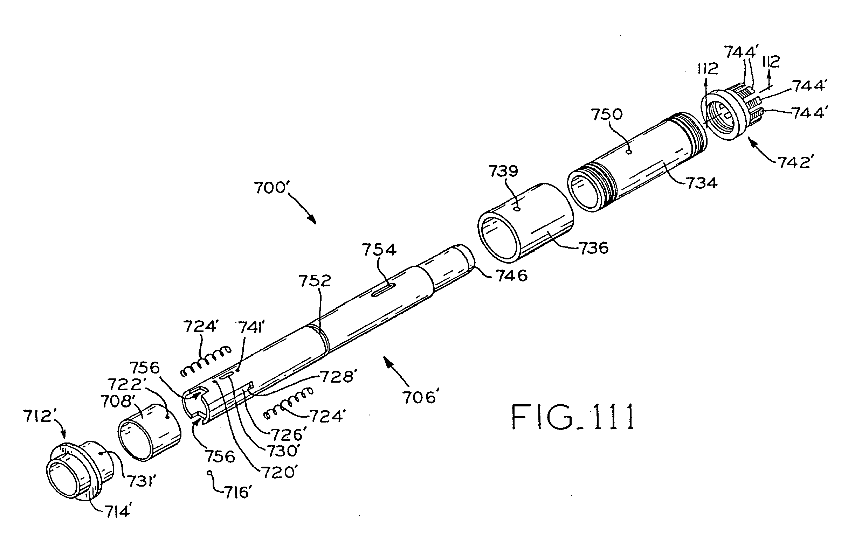

Referring to Figures 111-115, alternative embodiment unitube retractor 700' is illustrated. Unitube retractor 700' includes a ball detent retaining mechanism as described above with respect to unitube retractor 700, with corresponding parts denoted with primed reference numerals. The ball detent retaining mechanism of unitube retractor 700' is structured and operates substantially identical to the ball detent retaining mechanism described above with respect to unitube retractor 700 and, therefore, a detailed description of this mechanism will not now be repeated for the sake of brevity.

Unitube retractor 700' utilizes instrument alignment cutouts in unitube body 706 as opposed to the longer longituninal slot of unitube body 706. Also, collar 708' and plunger 712' do not include cutouts corresponding to instrument alignment cutouts in unitube body 706, unlike collar 708 and plunger 712 of unitube retractor 700. With this in mind, the instrument alignment tabs associated with the instruments to be positioned in unitube retractor 700' will not protrude past the exterior wall of unitube body 706'. Similar alignment tabs, could be used with unitube retractor 700, allowing use of plunger 712' and collar 708' with unitube 700. Similarly, plunger 712 and collar 708 could be used with unitube retractor 700' if the alignment tabs of the instruments to be inserted in unitube retractor 700' extend past the exterior wall of unitube body 706'. Unitube body 706' includes a pair of opposing instrument alignment cutouts allowing 180° of instrument realignment, which would necessitate a pair of corresponding cutouts in plunger 712 and collar 708, if used with unitube retractor 700'. If a pair of cutouts are required in the plunger and collar, then the plunger and collar will either be constructed in two pieces, or the cutouts will not run the entire length of the plunger and collar as do the cutouts of plunger 712 and collar 708 illustrated in Figures 107-109.

Unitube retractor 700' employs lock ring 742 to secure unitube retractor 700' in access 101 formed in femur 108 as described above. Lock ring 742 includes a number of expandable fingers 744 as illustrated in Figures 113-115. In use, unitube retractor 700' is inserted through incision 106 until fingers 744 abut greater trochanter 110. In this position, a surgeon will utilize tactile feedback to position the distal end of unitube retractor 700' in access 101 formed in greater trochanter 110. In one exemplary embodiment, a fluoroscope will be utilized to facilitate positioning of the distal end of unitube retractor 700' in access 101 formed in greater trochanter 110. After insertion of unitube retractor 700' into access 101 and alignment of instrument alignment cutouts 756 with an appropriate physiological landmark such as, the longitudinal axis of the femur, fingers 744 are expanded from the position illustrated in Figure 113 to the position illustrated in Figures 114 and 115 to secure unitube retractor 700' in femur 108. Figures 111 and 112 illustrate alternative embodiment lock ring 742' having teeth 748 radially extending from fingers 744 to facilitate locking of lock ring 742' in femur 108.

As illustrated in Figure 112, each finger 744' of lock ring 742' includes internal ramp 749. Although not illustrated, each finger 744 of lock ring 742 similarly includes an internal ramp. As illustrated in Figure 111, unitube body 706' includes beveled distal end 746. In the unactuated position of unitube retractor 700' as illustrated in Figure 113, beveled distal end 746 of unitube body 706' abuts internal ramps 749 of fingers 744. To actuate fingers 744 from the position illustrated in Figure 113 to the position illustrated in Figure 114 to effect locking of unitube retractor 700' to femur 108, unitube body 706' is longitudinally displaced toward lock ring 742, with beveled distal end 746 of unitube body 706' cooperating with internal ramps 749 of expandable fingers 744 to force expandable fingers 744 to move radially outwardly as illustrated in Figures 114 and 115.

A number of mechanisms may be employed to effect the necessary longitudinal displacement of unitube body 706' relative to lock ring 742. Figures 111, 113, and 114 illustrate one such mechanism. As illustrated in Figures 111, 113, and 114, threaded driver 736 is rotationally connected to unitube body 706' via set screw 738. Specifically, set screw 738 is threaded into set screw aperture 739 of threaded driver 736 and extends into annular threaded driver rotation groove 752 formed in unitube body 706'. In this way, threaded driver 736 may rotate relative to unitube body 706', but may not be longitudinally displaced relative to unitube body 706'. Connector shaft 734 is positioned about unitube body 706' and is threaded to threaded driver 736. After connector shaft 734 is positioned about unitube body 706', a set screw is threaded into set screw aperture 750 of connector shaft 734 and extends into guide slot 754 formed in unitube body 706' to restrict relative movement between connector shaft 734 and unitube body 706' to axial movement only. Connector shaft 734 is further threaded to lock ring 742, although, in an alternative embodiment, lock ring 742 could be secured to connector shaft 734 via any one of a number of connectors including, e.g., one or more set screws. In the position illustrated in Figure 113, connector shaft 734 is threaded into threaded driver a sufficient distance to place beveled distal end 746 (Figure 111) of unitube body 706' in abutting relationship with the internal ramps of expandable fingers 744 of lock ring 742. To actuate unitube retractor into the position illustrated in Figure 114, connector shaft 734 is held stationary, while threaded driver 736 is rotated to continue threading connector shaft 734 into threaded driver 736 and thereby force unitube body 706', which cannot be longitudinally displaced relative to threaded driver 736, further into lock ring 742, whereby beveled distal end 746 of unitube body 706' cooperates with internal ramps 749 of expandable fingers 744 to force expandable fingers 744 into the position illustrated in Figure 114. Specifically, set screw 738 acts against threaded driver rotation groove 752 to force unitube body 706' further into lock ring 742 as connector shaft 734 is threaded into threaded driver 736.

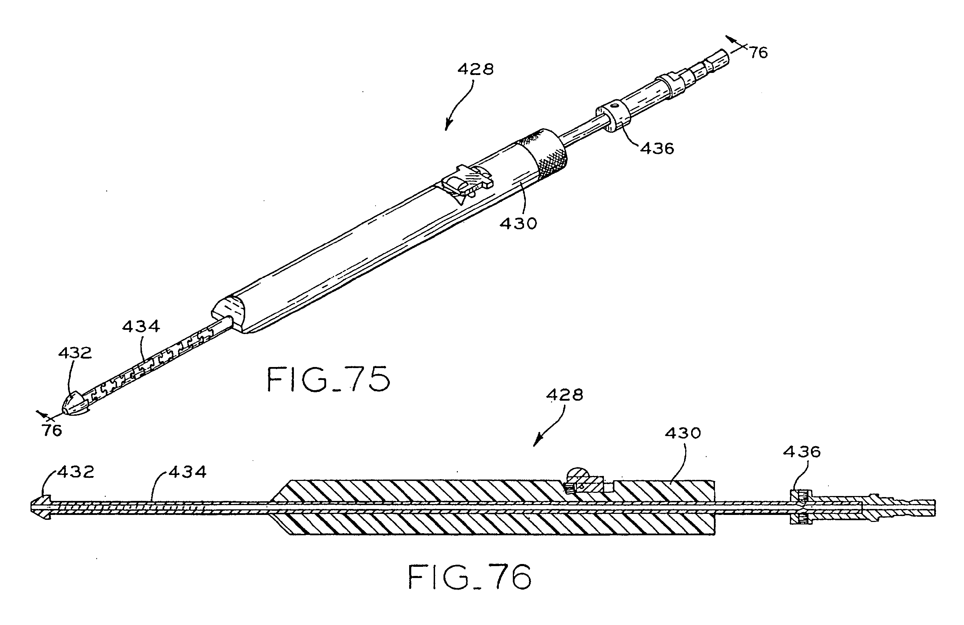

In an alternative embodiment of the present invention, flexible reamer 428 illustrated in Figures 75 and 76 is utilized in lieu of the curved reamers described above to ream into femoral head 114 and into the shaft of femur 108. As illustrated in Figures 75 and 76, flexible reamer 428 includes reaming head 432 and flexible reaming shaft 434. As illustrated in Figure 76, flexible reaming shaft 434 is canulated, allowing for insertion of flexible reamer shaft 434 over a guide wire to guide reaming into femoral head 114 and into the shaft of the femur 108. Flexible reamer 428 illustrated in Figures 75 and 76 utilizes flexible reamer guide tube 430 and a latch member associated with a particular reamer/guide tube of the present invention. However, flexible reamer 428 may include various guide tubes having physical characteristics allowing for use of flexible reamer 428 with the various guide tube/retractors of the present invention. As illustrated in Figures 75 and 76, the proximal end of flexible reamer shaft 434 is connected to flange 436 which acts against the proximal end of flexible reamer guide tube 430 to limit the reaming depth of flexible reamer 428.

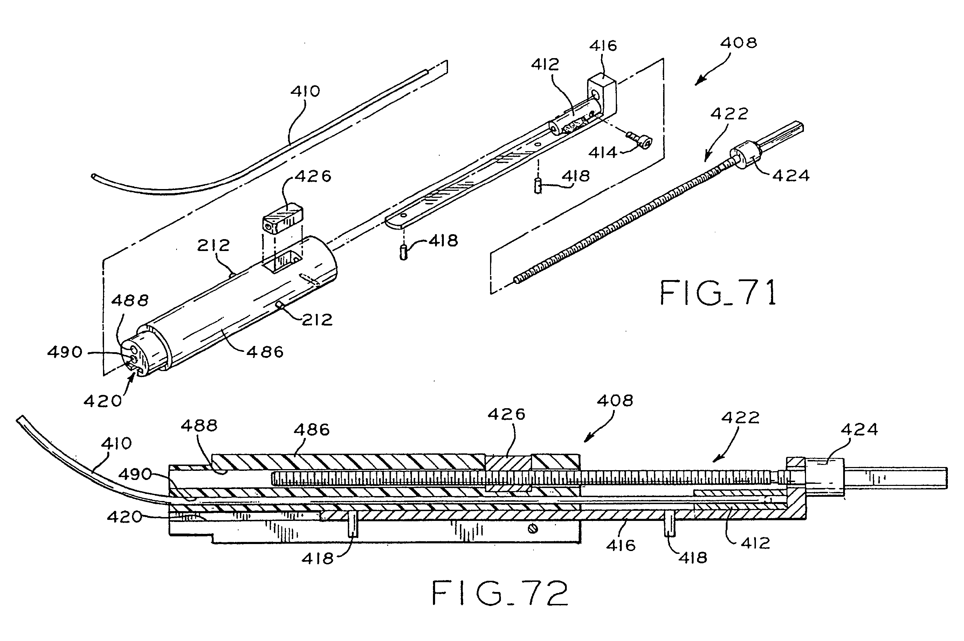

In one exemplary embodiment, flexible reamer guide 408 (Figures 71 and 72) is utilized to position guide wire 410 within the femur to guide flexible reamer 428. As illustrated in Figures 71 and 72, flexible reamer guide 408 includes guide 416 having guide shaft fixation channel 412 formed therein. Guide 416 is insertable within guide channel 420 of the main body of flexible reamer guide 408 as illustrated in Figure 72. Guide pegs 418 depend from guide 416 and are further inserted within guide channel 420 as illustrated in Figure 72. Flexible reamer guide tube 486 of flexible reamer guide 408 includes advance/retract screw aperture 488 and guide wire aperture 490. With guide 416 inserted in guide channel 420 of flexible reamer guide tube 486, guide wire 410 is inserted in guide wire aperture 490 and positioned within guide shaft fixation channel 412. Set screw 414 is utilized to secure guide wire 410 within guide shaft fixation channel 412. Advance/retract screw 422 traverses a proximal aperture in guide 416 and advance/retract screw aperture 488, and is threadably engaged with receiving block 426 as illustrated in Figure 72. Advance/retract screw 422 includes flange 424 for abutting the proximal end of guide 416 and for forcing guide 416 to be distally displaced in flexible reamer guide tube 486 in response to distal movement of advance/retract screw 422. Guide wire 410 is formed from a memory metal such as, e.g., NITINOL. With this in mind, advance/retract screw 422 may be retracted from receiving block 426 to allow guide wire 410 to retreat into guide wire aperture 490 to completely retract guide wire 410 within flexible reamer guide tube 486 of flexible reamer guide 408, without losing the ability of guide wire 410 to regain the bent shape illustrated in Figure 71.

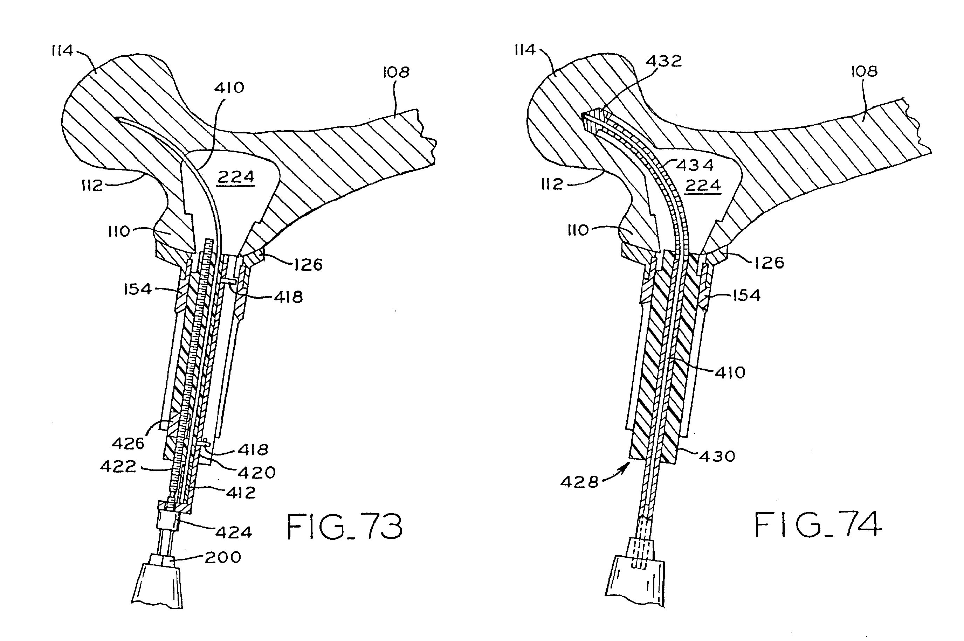

In use, flexible reamer guide 408 is inserted within a guide tube/retractor of the present invention with guide wire 410 not protruding from the distal end of guide wire aperture 490. The proximal end of advance retract screw 422 is thereafter actuated to force guide 416 and, consequently, guide wire 410 through flexible reamer guide tube 486 and into femoral head 414 as illustrated in Figure 73. Once guide wire 410 achieves the position illustrated in Figure 73, set screw 414 may be removed and flexible reamer guide 408 removed from the guide tube/retractor, leaving guide wire 410 in place within femur 108. Flexible reamer 428 may then be operably inserted in guide tube/retractor 154 as illustrated in Figure 74 and, with guide wire 410 positioned within the canula of flexible reamer 428, femoral cavity 224 may be extended into femoral head 114 as illustrated in Figure 74, with flexible reamer 428 being guided by guide wire 410. A similar technique may be utilized for advancing guide wire 410 into the femoral shaft to extend femoral cavity 224 therein.

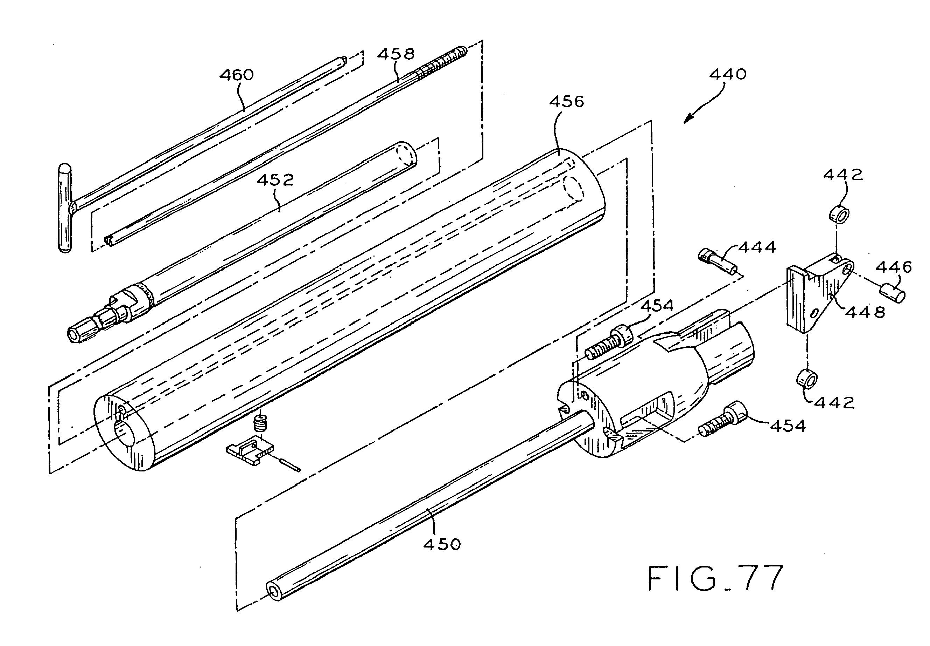

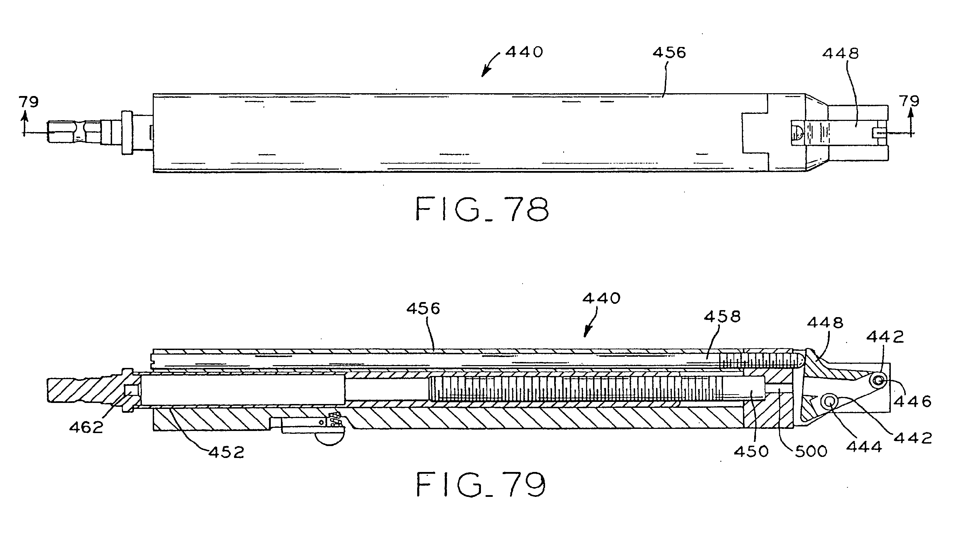

In a further alternative embodiment of the present invention, flexible reamer guide wire bender 440 as illustrated in Figures 77-79 is utilized to in vivo bend a guide wire to guide reaming into, e.g., femoral head 114 as illustrated, e.g., in Figure 73. As illustrated in Figures 77-79, flexible reamer guide wire bender 440 includes guide tube 456 for insertion into a guide tube/retractor of the present invention. Guide tube 456 includes a pair of elongate apertures. A first of these apertures accommodates inner wire tube 450 and outer wire tube 452 as illustrated, e.g., in Figure 79. The second of the elongate apertures formed in guide tube 456 accommodates adjustment screw 458 as illustrated, e.g., in Figure 79. Wire shaping head 448 is pivotally connected via pivot pin 444 to the distal end of flexible reamer guide wire bender 440 as illustrated in Figure 79. As illustrated in Figures 77 and 79, roller 442 is positioned about pivot pin 444. Wire shaping head 448 further includes roller pin 446 for connecting a second roller 442 in a rotatable manner to wire shaping head 448. As illustrated in Figure 77, screws 454 are utilized to affix the distal end of flexible reamer guide wire bender 440 to guide tube 456. As illustrated in Figure 79, outer wire tube 452 includes proximal wire extreme 462 against which an end of a guide wire will abut. Outer wire tube 452 is threadably engagable with either guide tube 456 or inner wire tube 450 so that outer wire tube 452 may be advanced into guide tube 456 to force a guide wire positioned against proximal wire extreme 462 through distal aperture 500 of flexible reamer guide wire bender 440. Adjustment screw 458 is utilized to rotate wire shaping head 448 about pivot pin 444 whereby rollers 442 bend a guide wire into the desired shape as it exits distal aperture 500 . Shaping of a guide wire in vivo with flexible reamer guide wire bender 440 may be observed with a fluoroscope.

A guide wire bent with flexible reamer guide wire bender 440 will be advanced into, e.g., femoral head 114 as illustrated, e.g., in Figure 73 with respect to guide wire 410. In this way, a flexible reamer will be utilized to extend femoral cavity 224 toward the femoral head as illustrated in Figure 74. A similar procedure may be utilized for extending femoral cavity 224 into the shaft of femoral 108.

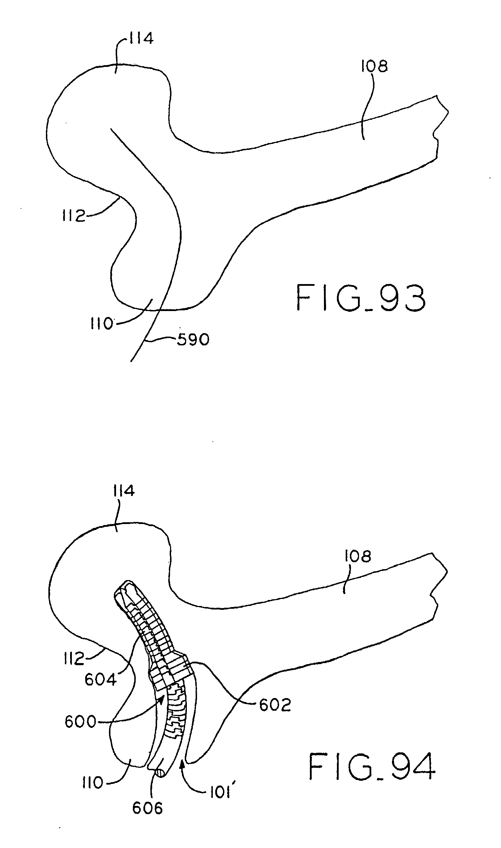

In yet another alternative embodiment of the present invention, flexible reamers having flexible reaming heads are utilized to form the cavity in femur 108 into which a femoral implant in accordance with the present invention is implanted. As illustrated in Figure 93, guide wire 590 is inserted into femur 108 and extends from greater trochanter 110, through femoral neck 112, and into femoral head 114. Guide wire 590 can be inserted into femur 108 utilizing flexible reamer guide 408 (Figures 71 and 72), or flexible reamer guide wire bender 440 (Figures 77-79). After inserting guide wire 590 into femur 108, flex up reamer 600 is used to ream a path from greater trochanter 110, through femoral neck 112, and into femoral head 114 as illustrated in Figure 94. In one embodiment of the present invention, access 101 is formed in femur 108 prior to using flex up reamer 600 to ream a path from greater trochanter 110, through femoral neck 112, and into femoral head 114. As illustrated in Figure 96, flex up reamer 600 includes elongate aperture 611. In use, guide wire 590 is positioned through elongate aperture 611 to guide reaming from greater trochanter 110, through femoral neck 112, and into femoral head 114.

As illustrated in Figures 94-96, flex up reamer 600 includes a reamer head having large diameter portion 602 and small diameter portion 604, with flexible cuts throughout the length of the flex up reamer head to allow the flex up reamer head to curve along the path defined by guide wire 590. A number of flexible cuts may be utilized along the length of the reamer head of flex up reamer 600, including the flexible cuts described in U.S. Patent No. 6,053,922 with respect to flexible reamer shafts. Flex up reamer 600 may be inserted through any of the guide tube/retractors of the present invention, and may include a cooperating guide tube matched to the guide tube/retractor utilized. Flex up reamer 600 advantageously includes large diameter portion 602 and small diameter portion 604 sized to form apertures accommodating lag screw tube 266, and lag screw shaft 274, respectively.

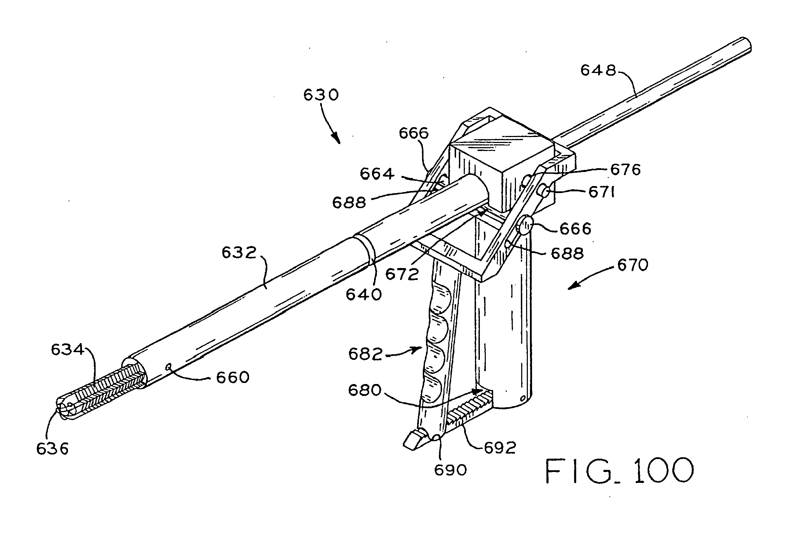

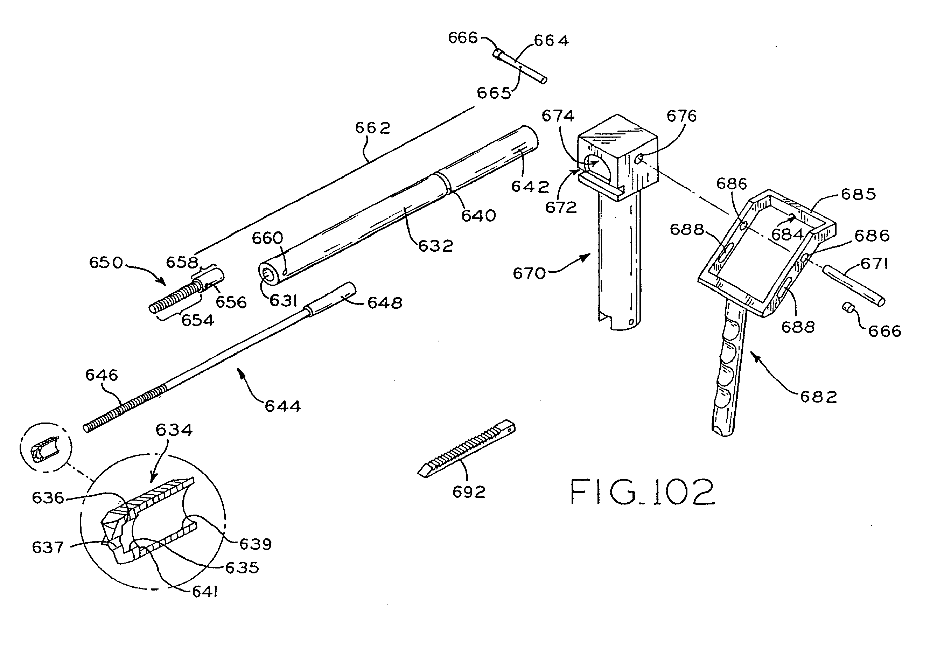

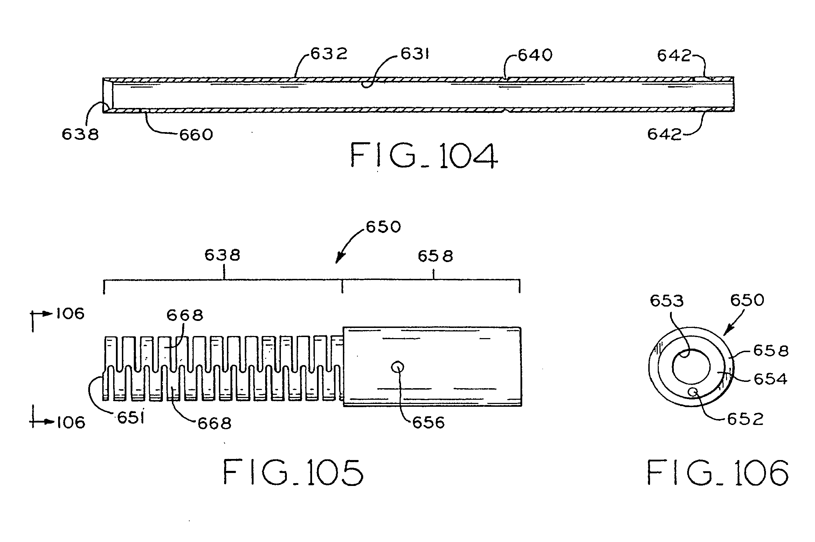

After formation of femoral head arm 256' (Figure 103) of the implant cavity, swivel/down reamer assembly 630 (Figures 100-102) is utilized to extend the implant cavity as illustrated in Figure 103. Referring to Figures 100-102, swivel/down reamer assembly 630 includes tool housing 632 having longitudinal aperture 631 running the length thereof as illustrated in Figure 104. Tool housing 632 includes detent groove 640 for receiving the ball detent of the ball detent retaining mechanism described above. Tool housing 632 further includes set screw aperture 660 for securing flexible guide shaft 650 therein. As illustrated in Figure 102, flexible guide shaft 650 includes set screw aperture 656 corresponding to set screw aperture 660 in tool housing 632.

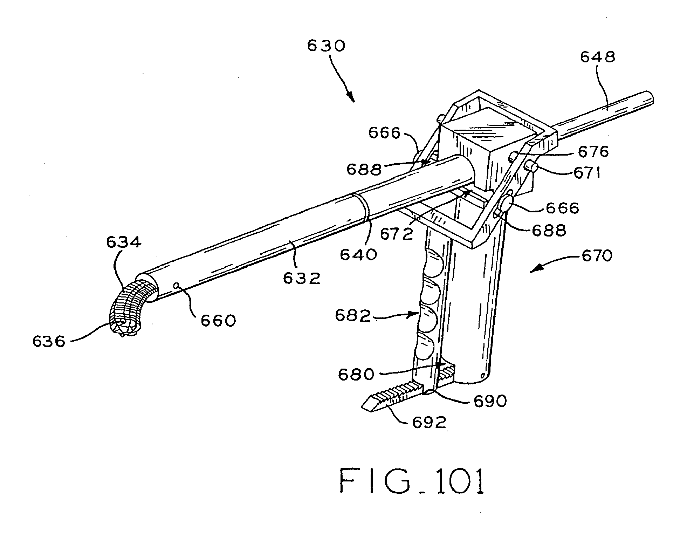

As illustrated in Figures 102 and 105, flexible guide shaft 650 includes flexible portion 654 and proximal end 658, with set screw aperture 656 formed in proximal end 658. Flexible portion 654 of flexible guide shaft 650 can be formed with a plurality of alternating, substantially semi-circular cuts 668 as illustrated in Figure 105. Specifically, cuts 668 are alternatively formed from the top and the bottom of flexible portion 654 as illustrated in Figure 105. As further illustrated in Figure 105, alternating cuts 668 overlap the center line of flexible guide shaft 650. Using non-continuous cuts as illustrated in Figure 105 to create flexibility in flexible portion 654 of flexible guide shaft 650 also limits flexibility to a plane perpendicular to the cuts because continuous material remains on either outside edge of flexible portion 654 of flexible guide shaft 650. This additional material at both sides of flexible guide shaft 650 advantageously prevents axial compression of the tube along the longitudinal axis thereof. In an alternative embodiment, cuts 668 are pie shaped, terminating in an apex toward the center of flexible portion 654 of flexible guide shaft 650. In construction, proximal end 658 of flexible guide shaft 650 is positioned within longitudinal aperture 631 of tool housing 632 and secured therein via a set screw. When proximal end 658 of flexible guide shaft 650 is secured within tool housing 632, flexible portion 654 of flexible guide shaft 650 protrudes from tool housing 632. Flexible guide shaft 650 includes reamer shaft aperture 653 (Figure 106) running the length thereof. Reamer shaft aperture 653 of flexible guide shaft 650 accommodates flex down reamer shaft 644 (Figure 102). Referring to Figure 102, to assemble swivel/down reamer assembly 630, flex down reamer shaft 644 is positioned within reamer shaft aperture 635 of flex down reamer head 634 and secured therein with a set screw positioned through set screw aperture 636 in flex down reamer head 634. Flexible guide shaft 650 is inserted through flexible guide shaft aperture 639 of flex down reamer head 634 until end 651 (Figure 105) of flexible guide shaft 650 abuts shoulder 641 (Figure 102) of flex down reamer head 634. Flex down reamer shaft 644 is positioned within reamer shaft aperture 653 of flexible guide shaft 650, with flexible guide shaft 650 positioned within flexible guide shaft aperture 639 of flex down reamer head 634. Flex down reamer shaft 644 extends the length of reamer shaft aperture 653 of flexible guide shaft 650 as well as the length of longitudinal aperture 631 of tool housing 632, with chuck end 648 of flex down reamer shaft 644 extending out of tool housing 632 as illustrated in Figures 100 and 101.

Prior to securing flexible guide shaft 650 to tool housing 632, and positioning flex down reamer shaft 644 therein, cable 662 is inserted through cable aperture 652, which runs the length of flexible guide shaft 650. After inserting cable 662 through cable aperture 652, a piece of material larger in cross sectional area than cable aperture 652 is secured to the end of cable 662 extending outwardly from end 651 of flexible guide shaft 650 to prevent cable 662 from being pulled out of cable aperture 652 in a distal to proximal direction relative to flexible guide shaft 650. In one exemplary embodiment, a ball of weld material is welded to the end of cable 662. In construction, cable 662 extends from flexible guide shaft 650 through the length of tool housing 632.

As illustrated in Figures 100 and 101, cable rod 664 traverses aligned cable rod slots 642 (Figures 102 and 104) formed in opposing sides of tool housing 632. Cable rod 664 includes cable aperture 665 for receiving cable 662. After cable 662 is inserted through cable aperture 665 in cable rod 664, the slack in cable 662 is eliminated and cable 662 is secured to cable rod 664. As illustrated in Figures 100-102, handle 670 includes cable rod cutout 672 accommodating cable rod 664. Handle 670 further includes tool housing aperture 674 into which tool housing 632 is positioned. Tool housing 632 can be secured to handle 670 via a set screw or other fastener extending through handle 670 into tool housing aperture 674.

As illustrated in Figures 100 and 101, lever handle 682 is pivotally connected to handle 670 via pivot shaft 671, with pivot shaft 671 traversing pivot apertures 686 and 676 (Figure 102) in lever handle 682 and handle 670, respectively. Lever handle 682 includes a pair of elliptical cable rod apertures 688 in opposing arms thereof. Elliptical cable rod apertures 688 accommodate cable rod 664. With cable rod positioned through elliptical cable rod apertures 688 in lever handle 682, cable rod end nuts 666 are secured to opposing ends of cable rod 664 to prevent axial displacement of cable rod 664. To complete assembly of swivel/down reamer assembly 630, ratchet bar 692 is positioned within ratchet cutout 680 of handle 670 and pivotally connected thereto, with a leaf spring interposed between ratchet bar 692 and handle 670 to bias ratchet bar 692 upwardly toward handle 670. As illustrated in Figures 100 and 101, lever handle 682 includes pawl end 690 for engaging the ratchet teeth of ratchet bar 692.

In use, swivel/down reamer assembly 630 can be actuated from a straight or unflexed position as illustrated in Figure 100 to a flexed position as illustrated in Figure 101. To actuate swivel/down reamer assembly 630 from the straight position illustrated in Figure 100 to the flexed position illustrated in Figure 101, force is applied to lever handle 682 to pivot lever handle 682 about pivot shaft 671 toward handle 670. When lever handle 682 is actuated in this manner, cable rod 664 is pulled toward handle 670, causing flexible guide shaft 650 to flex downwardly. Specifically, cable 662 pulls the lower portion of flexible guide shaft inwardly, flexing flexible guide shaft 650 whereby the top portion of flexible guide shaft 650 is placed in tension or stretches, and the bottom portion of flexible guide shaft 650 is compressed. As illustrated in Figures 100-102, flex down reamer head 634 includes flexible cuts along its length. When flexible guide shaft 650 flexes as described above, flex down reamer head 634 similarly flexes downwardly, as flex down reamer shaft is positioned within flexible guide shaft aperture 639 of flex down reamer head 634 when swivel/down reamer assembly 630 is actuated from the straight position illustrated in Figure 100 to the flexed position illustrated in Figure 101. As illustrated in Figure 101, pawl end 690 of lever handle 682 engages the teeth of ratchet bar 692 to retain swivel/down reamer assembly 630 in the actuated position of Figure 100. As described above, ratchet bar 692 is biased toward handle 670 by a leaf spring. To release swivel/down reamer assembly 630 from the actuated position illustrated in Figure 100, a distal end of ratchet bar 692 may be pushed downwardly, i.e., away from handle 670 to release pawl end 690 of lever handle 682 from engagement with the teeth of ratchet bar 692.

Referring to Figure 102, lever handle 682 includes radiused cutout 684 sized to accommodate flex down reamer shaft 644. In the straight or unflexed position illustrated in Figure 100, radiused cutout 684 is positioned about flex down reamer shaft 644 such that cross bar 685 of lever handle 682 abuts the shoulder formed on flex down reamer shaft 644 between chuck end 648 and the remainder of flex down reamer shaft 644. This cooperating shoulder arrangement prevents flex down reamer shaft 644 and, consequently, flex down reamer head 634 from being advanced through and away from tool housing 632. When swivel/down reamer assembly 630 is actuated into the flexed position as illustrated in Figure 101, lever handle 682 is moved so that flex down reamer shaft 644 is no longer positioned within radiused cutout 684 contacting flex down reamer shaft 644 and the cooperating shoulder arrangement which prevents flex down reamer shaft 644 and flex down reamer head 634 from being advanced through tool housing 632 is eliminated.

In use, flex down reamer head 634 is inserted into access 101' formed in femur 108 as described above. As illustrated in Figure 103, on initial insertion, flex down reamer head 634 is positioned about flexible guide shaft 650 as illustrated in Figure 103. As illustrated in Figure 103, tool housing 632 abuts greater trochanter 110 when swivel/down reamer assembly 630 is utilized to extend implant cavity 224' as illustrated in Figure 3. Upon insertion of flex down reamer head 634 through access 101' in femur 108, flex down reamer head 634 is actuated by coupling an actuation device to chuck end 648 of flex down reamer shaft 644 and supplying rotational motion thereto. With flex down reamer head 634 rotating to ream bone from femur 108, swivel/down reamer assembly is actuated from the straight or non-flexed positioned illustrated in Figure 100 to the flexed position illustrated in Figure 101 to extend implant cavity 224 from femoral head arm 256' formed by flex up reamer 600, as illustrated in Figure 94, toward the shaft of femur 108. Actuation of swivel/down reamer assembly 630 from the straight or non-flexed position illustrated in Figure 100 to the flexed position in Figure 101 generally effects a swivel type reaming as described above. After swivel reaming is complete, chuck end 648 of flex down reamer shaft 644 is advanced through tool housing 632 to advance flex down reamer head 634 into and through the intramedullary canal of femur 108. As flex down reamer head 634 is advanced relative to tool housing 632, flex down reamer head 634 is also advanced relative to flexible guide shaft 650 so that flexible reamer head 634 is eventually moved out of engagement with flexible guide shaft 650, i.e., flexible guide shaft 650 is no longer positioned within flexible guide shaft aperture 639 of flex down reamer head 634 (see Figure 102). As flex down reamer head 634 is advanced toward the intramedullary canal of femur 108, flex down reamer head 634 will be directed into the intramedullary canal of the femur as it is moved from engagement with flexible guide shaft 650 due to the curvature provided by flexible guide shaft 650 and also due to the softer cancellous bone occupying the intramedullary canal versus the harder cortical bone material of the femur. To facilitate appropriate movement of flex down reamer head 634 into the intramedullary canal of femur 108, flex down reamer head 634 has a generally bullet shape as illustrated, e.g., in Figures 100-103. The distal end of bullet shaped flex down reamer head 634 will glance off the harder cortical wall of the femur and be directed into the intramedullary canal as described above.

While this invention has been described as having exemplary designs, the present invention may be further modified with the spirit and scope of this disclosure. This application is therefore intended to cover any variations, uses, or adaptations of the invention utilizing its general principles. Further, this application is intended to cover such departures from the present disclosure as come within known or customary practice in the art to which this invention pertains.

## Claims

1. A prosthetic implant, comprising:
an acrylic bag;
a fill access providing access to an interior of said bag; and
a tube, said bag secured to an exterior of said tube.

2. The prosthetic implant of Claim 1, further comprising:
a lag screw, comprising:
a shaft, said shaft having an exterior geometry cooperating with an interior geometry of said tube to allow relative axial displacement of said lag screw and said tube and to resist relative rotational displacement of said lag screw and said tube; and
a screw head, said screw head rotabably connected to said shaft.

3. The prosthetic implant of Claim 2, wherein said lag screw shaft includes an interior elongate aperture, and wherein said screw head includes a drive connector for selective engagement with a rotatable drive instrument, said interior elongate aperture of said lag screw shaft providing access to said drive connector.

4. The prosthetic implant of Claim 2, wherein said lag screw shaft is curved from a proximal end thereof to a distal end thereof, and wherein said tube is curved from a proximal end thereof to a distal end thereof, said tube and said lag screw shaft having a similar curvature, whereby said lag screw shaft is slidable relative to said tube when said lag screw shaft is placed within said tube.

5. The prosthetic implant of Claim 1, further comprising a fill tube, said fill tube providing said access.

6. The prosthetic implant of Claim 5, wherein said fill tube has a length, said length sized to allow retrograde filling of said bag.

7. The prosthetic implant of Claim 1, further comprising:
a substantially V-shaped expansion bar positioned within said bag.

8. A reamer for forming a cavity in a bone, comprising:
a rotatable reamer shaft having proximal and distal ends; and
a reamer head coupled to said distal end of said rotatable reamer shaft, said reamer head having an exterior, said exterior of said reamer head having a plurality of flutes defining a plurality of blades therebetween, said reamer head having reamer head flexing means for allowing said reamer head to flex, said reamer head having a length measured from a distal end to a proximal end thereof.

9. The reamer of Claim 8, wherein said reamer head flexing means comprises at least one cut formed in said reamer head.

10. The reamer of Claim 9, wherein said at least one cut comprises a helical cut running the length of said reamer head.

11. The reamer of Claim 8, wherein said exterior of said reamer head has at least two differently sized diameters defining at least two reaming diameters.

12. The reamer of Claim 8, wherein said distal end of said rotatable reamer shaft includes shaft flexing means for allowing said distal end of said rotatable reamer shaft to flex, said distal end of said rotatable reamer shaft having a length.

13. The reamer of Claim 12, wherein said shaft flexing means comprises at least one cut formed in said distal end of said rotatable reamer shaft.

14. The reamer of Claim 13, wherein said at least one cut comprises a helical cut running the length of said distal end of said rotatable reamer shaft.

15. A reamer for forming a curved cavity in a bone, comprising:
a rotatable reamer shaft having proximal and distal ends; and
a reamer head coupled to said distal end of said rotatable reamer shaft, said reamer head having an exterior, said exterior of said reamer head having a plurality of flutes defining a plurality of blades therebetween, said reamer head having at least one cut formed therein, said cut intersecting at least two blades, said reamer head having a length measured from a distal end to a proximal end thereof.

16. The reamer of Claim 15, wherein said at least one cut comprises a helical cut running the length of said reamer head.

17. The reamer of Claim 15, wherein said exterior of said reamer head has at least two differently sized diameters defining at least two reaming diameters.

18. The reamer of Claim 15, wherein said distal end of said rotatable reamer shaft includes a helical cut extending from said distal end of said rotatable reamer shaft to said proximal end of said rotatable reamer shaft.

19. In combination, a reamer for forming a cavity in bone, and a reamer guide for controlling a radius of curvature of the reamer, comprising:
a reamer, comprising:
a rotatable reamer shaft having proximal and distal ends, said distal end of said rotatable reamer shaft including shaft flexing means for allowing said distal end of said rotatable reamer shaft to flex; and
a reamer head coupled to said distal end of said rotatable reamer shaft, said reamer head having an exterior, said exterior of said reamer head having a plurality of flutes defining a plurality of blades therebetween, said reamer head having reamer head flexing means for allowing said reamer head to flex, said reamer head having a length measured from a distal end to a proximal end thereof, said reamer head having a flexible guide shaft aperture extending from the proximal end of said reamer head toward the distal end thereof; a reamer guide, comprising:
a tool housing having a longitudinal aperture;
a guide shaft positioned in said longitudinal aperture of said tool housing and coupled to said tool housing, said guide shaft having a longitudinal aperture, with a guide shaft wall defined between an exterior of said guide shaft and said longitudinal aperture, said reamer shaft occupying said longitudinal aperture, said guide shaft having proximal and distal ends, said distal end of said guide shaft having guide shaft flexing means for allowing said guide shaft to flex, said distal end of said guide shaft positioned within said flexible guide shaft aperture extending from the proximal end of said reamer head toward the distal end thereof; and
actuation means for actuating said guide shaft into a flexed position.

20. The combination of Claim 19, wherein said actuation means for actuating said guide shaft into a flexed position comprises:
a longitudinal wall bore formed in said wall of said guide shaft;
a cable positioned in said wall bore of said guide shaft, said cable having a distal end of larger size than said wall bore, whereby said cable cannot be pulled from said wall bore in a distal to proximal direction relative to said guide shaft; and
pulling means for pulling said cable in a distal to proximal direction relative to said guide shaft, whereby pulling said cable causes flexure of said guide shaft.

21. The combination of Claim 20, wherein said pulling means for pulling said cable in a distal to proximal direction relative to said guide shaft comprises:
a handle, said tool housing coupled to said handle;
a lever handle pivotally connected to said handle, said cable connected to said lever handle, whereby pivoting of said lever handle about said handle pulls said cable in a distal to proximal direction relative to said guide shaft, said lever handle having an initial position in which said cable is not pulled by said lever handle.

22. The combination of Claim 21, wherein said lever handle includes an advance prevention means for selectively preventing said reamer shaft from being advanced through said tool housing.

23. The combination of Claim 22, wherein said advance prevention means comprises:
a lever handle shoulder formed on said lever handle; and
a reamer shaft shoulder formed on said rotatable reamer shaft, said lever handle shoulder abutting said reamer shaft shoulder when said lever handle maintains said initial position.

24. The combination of Claim 19, wherein said shaft flexing means for allowing said distal end of said rotatable reamer shaft flex comprises at least one cut formed in said distal end of said rotatable reamer shaft.

25. The combination of Claim 24, wherein said at least one cut comprises a helical cut.

26. The combination of Claim 19, wherein said reamer head flexing means comprises at least one cut formed in said reamer head.

27. The combination of Claim 19, wherein said at least one cut comprises a helical cut running the length of said reamer head.

28. The combination of Claim 19, wherein said guide shaft flexing means comprises at least one cut formed in said guide shaft.

29. The combination of Claim 28, wherein said at least one cut in said guide shaft comprises a helical cut running the length of said distal end of said rotatable reamer shaft.

30. The combination of Claim 28, wherein said at least one cut comprises a plurality of substantially semi-circular cuts oriented substantially perpendicularly to a longitudinal axis of said distal end of said guide shaft, said guide shaft having a top and a bottom, each said substantially semi-circular cut formed through one of said top and said bottom of said guide shaft, whereby adjacent ones of said substantially semi-circular cuts are alternatively formed in said top and said bottom of said guide shaft, said substantially semi-circular cuts running the length of said distal end of said guide shaft.

31. In combination, a reamer for forming a curved cavity in bone, and a reamer guide for controlling a radius of curvature of the reamer, comprising:
a reamer, comprising:
a rotatable reamer shaft having proximal and distal ends, said rotatable reamer shaft having a flexible reamer shaft portion extending from said distal end toward said proximal end; and
a reamer head coupled to said distal end of said rotatable reamer shaft, said reamer head having an exterior, said exterior of said reamer head having a plurality of flutes defining a plurality of blades therebetween, said reamer head being flexible along the entire length thereof;
a reamer guide, comprising:
a tool housing having a longitudinal aperture;
a guide shaft positioned in said longitudinal aperture of said tool housing and coupled to said tool housing, said guide shaft having a longitudinal aperture, with a guide shaft wall defined between an exterior of said guide shaft and said longitudinal aperture, said reamer shaft occupying said longitudinal aperture of said guide shaft, said guide shaft having proximal and distal ends, said guide shaft having a guide shaft flexible portion extending from said distal end of said guide shaft toward said proximal end of said guide shaft, said distal end of said guide shaft positioned within a flexible guide shaft aperture extending from the proximal end of said reamer head toward the distal end thereof.

32. The combination of Claim 31, further comprising:
a longitudinal wall bore formed in said wall of said guide shaft;
a cable positioned in said wall bore of said guide shaft, said cable having a distal end of larger size than said wall bore, whereby said cable cannot be pulled from said wall bore in a distal to a proximal direction relative to said guide shaft;
a handle, said tool housing coupled to said handle;
a lever handle pivotally connected to said handle, said cable connected to said lever handle, whereby pivoting of said lever handle about said handle pulls said cable in a distal to proximal direction relative to said guide shaft, said lever handle having an initial position in which said cable is not pulled by said lever handle.

33. The combination of Claim 32, further comprising:
a lever handle shoulder formed on said lever handle; and
a reamer shaft shoulder formed on said rotatable reamer shaft, said lever handle shoulder abutting said reamer shaft shoulder when said lever handle maintains said initial position.
